# EUROPEAN PATENT SPECIFICATION

(11) **EP 0 665 743 B1**
(45) Date of publication and mention of the grant of the patent: **12.12.2001**
(21) Application number: 93924329.1
(22) Date of filing: 13.10.1993
(51) Int. Cl.: A61K 9/127, A61L 2/02

(54) **INTERDIGITATION-FUSION LIPOSOMES AND GELS**
INTERDIGITATION-FUSIONSLIPOSOMEN UND GELS
LIPOSOMES ET GELS OBTENUS PAR INTERDIGITATION-FUSION

(30) Priority: 14.10.1992 US 961277; 24.05.1993 US 66539
(43) Date of publication of application: 09.08.1995
(73) Proprietor: THE LIPOSOME COMPANY, INC., Princeton, NJ 08540 (US)
(72) Inventor: BONI, Lawrence, T., Monmouth Junction, NJ 08852 (US); JANOFF, Andrew, S., Yardley, PA 19067 (US); MINCHEY, Sharma, R., Monmouth Junction, NJ 08852 (US); PERKINS, Walter, R., Monmouth Junction, NJ 08852 (US); SWENSON, Christine, E., Princeton Junction, NJ 08550 (US); AHL, Patrick, L., Princeton, NJ 08540 (US); DAVIS, Thomas, S., Valhalla, NY 10595 (US)
(74) Representative: Warcoin, Jacques
(86) International application number: US9309878
(87) International publication number: WO9408565

(56) References cited:
- EP-A- 0 190 050
- WO-A-91/10422
- VMT VOEDINGSMIDDELENTECHNOLOGIE vol. 24, no. 24 , 21 November 1991 , ZEIST (NL) pages 11 - 14 XP233531 B.A.A. MERTENS 'toepassing van hoge isostatische druk'
- BIOCHEMISTRY vol. 22, no. 2 , 18 January 1983 , EASTON (US) pages 409 - 415 P. L.-G. CHONG ET AL. 'a differential polarized phase fluorometric study of the effects of high hydrostatic pressure upon the fluidity of cellular membranes'

## Description

### Related U.S. Application Data

This application is a continuation-in-part of application Serial No. 07/961,277, filed October 14,1992, which is a continuation-in-part of application Serial No. 07/664,576, filed March 5, 1991, now abandoned, which is a continuation-in-part of application Serial No. 07/464,528, filed January 12, 1990, now abandoned.

### Field of the Invention

This invention relates to interdigitation-fusion (IF) liposomes and gels. These liposomes and gels capture high solute to lipid ratios. The term solute encompasses bioactive agents, including contrast agents. This invention also relates to the discovery of interdigitation of lipids to produce IF gels and liposomes, and the further discovery that such interdigitation to form liposomes according to the present invention is size dependent.

The present invention relates to a method for producing IF liposomes and gels. In the method of the present invention, liposomes formed by sonication, extrusion or alternative size reduction processes such as homogenization to the appropriate size are fused in the presence of a suitable inducer. This process produces a composition of the present invention in gel form. The gel itself can be employed, for example, for delivery of bioactive agents, or can be used to form IF liposomes, which in turn possess very high internal volumes and encapsulate large amounts of solute.

To produce IF liposomes from the gels, the gels are incubated at a temperature usually but not necessarily above the transition temperature (Tm) of the lipid used, such that liposomes are formed. The temperature required by the methods of the invention is that temperature for any given mixture of lipid, solute and inducer that induces a change in the material properties of the mixture thereby producing the IF liposomes of the invention. The liposomes formed from IF gels are IF liposomes. Preferably, but not necessarily, the inducer is also removed during the incubation step. The result is a composition comprising liposomes containing high solute to lipid ratios.

### Background of the Invention

The therapeutic properties of many drugs may be dramatically improved by the administration in a liposomally encapsulated form [See, for example P.N. Shek and R.F. Barber, Mod. Med. Canada, 41, 314-382, (1986)]. In certain cases, for example, in the administration of amphotericin B and doxorubicin [Lopez-Berestein, et al., J. Infect. Dis., 151, 704-710, (1985) and Rahman, et al., Cancer Res., 40, 1532 (1980)] toxicity is reduced while efficacy is maintained or even increased. The benefit obtained from liposomally encapsulated agents may be fortuitous, and likely results from the altered pharmacokinetics and biodistribution of the entrapped drug. [Ostro, et al., Amer. J. Hosp. Pharm., Vol. 46 Aug 1989]

A number of methods are presently available for "charging" liposomes with bioactive agents. For example, in a liposome-drug delivery system, a bioactive agent such as a drug may be entrapped in the liposome and then administered to the patient to be treated. See, for example, Rahman et al., U.S. Patent No. 3,993,754; Sears, U.S. Patent No. 4,145,410; Papahadjopoulos, et al., U.S. Patent No. 4,235,871; Lenk et al., U.S Patent No. 4,522,803; and Fountain et al., U.S. Patent No. 4,588,578. In addition to this basic method for entrapping a bioactive agent, if the bioactive agent is lipophilic, it may associate with the lipid bilayer. However, many of the pharmaceutical formulations produced utilizing the traditional methods suffer from the disadvantage of low drug to lipid ratio, scaleup problems and the use of toxic solvents.

In addition to the above-described methods, numerous bioactive agents have been shown to accumulate in liposomes in response to an imposed proton or ionic gradient known as "remote loading" [See, for example Mayer, et al., Biochim. Biophys. Acta, 857, 123, (1986), Mayer, et al., Biochemistry, 27, 2053, (1988) and M.B. Bally, et al., Chem. Phys. Lipids, 47, 97, (1988)]. This loading technique allows independent variation of any of the liposomal parameters. Much higher drug to lipid ratios can be achieved in comparison to conventional techniques [Mayer, et al. Chem. Phys. Lipids, 40, 333 (1986)]. The procedures and materials for remote loading are disclosed in Bally et al., U.S. Patent No. 5,077,056, issued December 31, 1991, and Mayer et al., U.S. Serial No. 07/636,015, filed January 4, 1991.

Liposomes are completely closed lipid bilayer membranes which contain entrapped aqueous volume. Liposomes may be unilamellar (single bilayer membrane) or multilamellar (onion-like structures characterized by multiple bilayer membranes, each separated from the next by an aqueous layer). The bilayer is composed of two lipid monolayers having a hydrophobic "tail" region and a hydrophilic "head" region. In the membrane bilayer, the hydrophobic (nonpolar) "tails" of the lipid monolayers orient toward the center of the bilayer, whereas the hydrophilic (polar) "heads" orient toward the aqueous phase. The basic structure of liposomes may be made by a variety of techniques known in the art.

One class of liposomes that may be used in the practice of the invention are those characterized as having substantially equal lamellar solute distribution. This class of liposomes is denominated as stable plurilamellar vesicles (SPLV) as defined in U.S. Patent No. 4,522,803 to Lenk, et al., monophasic vesicles as described in U.S. Patent No. 4,588,578 to Fountain, et al., and frozen and thawed multilamellar vesicles (FAT MLV) wherein the vesicles are exposed to at least one freeze and thaw cycle; this procedure is described in Bally et al., U.S. Patent No. 4,975,282, issued December 4, 1990, entitled "Multilamellar Liposomes Having Improved Trapping Efficiencies".

Liposomal encapsulation could potentially provide numerous beneficial effects for a wide variety of pharmaceutical agents and a high drug to lipid ratio should prove important in realizing the potential of liposomally encapsulated agents. The use of liposomes to administer drugs has raised problems with regard both to drug encapsulation and drug release during therapy. For example, there is a continuing need to increase drug to lipid ratios so as to minimize the lipid load presented to the patient.

Interdigitation of lipids is a phenomenon which has been recently explored in considerable detail by James L. Slater and Ching-Hsien Huang in Progress Lipid Res., 27, 325-359, 1988. In general, the art describes the interdigitation of various lipid species resulting from either the presence of various inducers and/or acyl chain length asymmetry (See Figures 1A and 1B). WO 91/10422 relates to a method for interdigitating-fusing sized liposomes. There has been no report in the literature, however, of the size dependency for fusing liposomes during interdigitation to produce the IF gels and liposomes of the present invention.

### Objects of the Present Invention

It is an object of the present invention to provide interdigitation-fusion gels and liposomes, which may be used for delivering solute in a number of applications, including therapeutic applications.

It is another object of the present invention to provide an interdigitation-fusion gel which contains saturated lipid and may additionally contain non-saturated lipids and effective concentrations of bioactive agents for formulation into compositions for topical or oral administration to a mammal, including humans.

It is a further object of the present invention to provide a method for producing interdigitation-fusion liposomes and gels which accumulate high concentrations of bioactive agents.

It is yet another object of the present invention to provide pharmaceutical compositions based upon the interdigitation-fusion gels and liposomes of the present invention.

It is still another object of the present invention to provide methods for making the interdigitation gels and IF liposomes of the present invention.

It is another object of the present invention to provide a novel method for trapping bioactive agents.

These and other objects of the present invention may be readily understood from the detailed description of the invention which is set forth herein.

### Summary of the Invention

The present invention relates to interdigitation-fusion (IF) liposomes and gels which can contain solute. These liposomes and gels capture high solute to lipid ratios, including bioactive agent. These IF gels and liposomes find use in a number of applications, including cosmetic, pharmaceutical and agricultural applications. In combination with bioactive agents, these liposomes and gels may be administered topically or systemically to plants and animals, especially mammals, including humans. In addition to the above applications, the IF gels and liposomes of the present invention are also useful in combination with resin technology, in particular, paint technology.

The compositions of the present invention comprise a sized liposome, preferably about 0.4 µm (microns) in diameter or less, more preferably about 0.05 µm (microns) in diameter or less, and most preferably about 0.025 µm (microns) in diameter, in combination with a solute, for example a bioactive agent and an amount of an interdigitation inducer effective to fuse the liposomes to produce an IF gel. The initial liposomes may alternatively be FAT MLVs. IF liposomes may be produced from the IF gels of the present invention. Preferably, in the IF liposomes and gels of the present invention, a saturated lipid, for example, dipalmitoylphosphatidylcholine (DPPC), dimyristoylphosphatidylcholine (DMPC), di-0-hexadecylphosphatidylcholine and distearoylphosphatidylcholine, as well as lipids in which the unsaturated carbon-carbon double bonds in the acyl side chains of the lipid are in the trans configuration, such as transdielaidoylphosphatidylcholine and dipalmitelaidoylphosphatidylcholines, or mixed fatty acid lipids such as palmitoyloleoylphosphatidylcholine (POPC) or 1-stearoyl-2-oleoyl phosphatidylcholine (SOPC), as well as other unsaturated lipids, may also be used.

In certain embodiments, unsaturated lipids can be employed in combination with the saturated lipids of the invention. It is generally preferred that when DOPC is the unsaturated lipid employed, it be used with the saturated lipid DPPC in no more than a proportion of 50 mole percent unsaturated lipid.

The interdigitation-fusion gel that is produced when sized liposomes are fused, preferably in the presence of an inducer, which may or may not contain a bioactive agent, may be used without further modification, or alternatively, the gel may be further modified, for example, by usually but not necessarily heating to a temperature above the lipid transition temperature (Tm) but in any case incubating the mixture to a temperature which will induce a change in the material properties of the mixture and thus induce the formation of IF liposomes from the IF gels of the invention, and further possibly removing the interdigitation inducer contained therein, to produce IF liposomes.

The IF liposomes of the present invention may be used to capture surprisingly high solute to lipid ratios, including bioactive agents. These IF liposomes may be used without further modification or they may be further sized to produce liposomes of varying and/or homogeneous size using techniques and methodologies readily available in the art, and which will be reviewed hereinbelow.

In the present invention, the preferred interdigitation inducer is a short-chain alcohol, such as those having 1 to 4 carbon atoms, preferably ethanol because of the ease with which it can be removed to produce IF liposomes from the IF gels of the present invention. However, any inducer that produces a fused IF gel from sized liposomes may be used in embodiments of the present invention.

Exemplary inducers for use in the present invention include, for example, polyols such as glycerol, ethylene glycol, short-chain alcohols such as methanol, ethanol, propanol, isopropanol and n-butanol and anesthetics such as chlorpromazine, tetracaine, phenylethanol, benzyl alcohol and phenylbutanol, and others including polymixin, myelin basic protein, choline, acetylcholine, Tris buffer and chaotropic salts such as, for example, thiocyanate. Ethanol, however, is preferred because of its ease of removal and pharmaceutical compatibility. The amount of inducer used in the present invention comprises an amount effective for producing interdigitation-fusion gels from sized liposomes. It is to be noted that in certain embodiments of the present invention the saturated lipid used to make IF gels and liposomes of the present invention may be a self-inducer, i.e., the saturated lipid will produce IF gels and liposomes of the present invention without the need to add an inducer. In particular, the use of di-0-hexadecylphosphatidylcholine (DHPC) in this aspect of the present invention as exemplary, is noted. Alternatively or additionally, as noted herein, a solute which may be a bioactive agent may itself also be an inducer.

In another embodiment of the present invention, hydrostatic pressure may be used as the interdigitation fusion inducer. In such cases, small liposomes are caused to form IF gels by the application of hydrostatic pressure. In particular embodiments of this aspect of the present invention, small liposomes comprising DPPC or DSPC can be induced by pressure to interdigitate, thus forming intermediary gels that form liposomes upon heating of the lipid above its phase transition temperature.

As a further aspect of this embodiment of the present invention, high hydrostatic pressures may be used to kill bacteria and to sterilize liposomal preparations. Thus, hydrostatic pressure can be used not only to induce interdigitation fusion, and but also to sterilize the resultant liposome preparations.

The IF liposomes and gels of the present invention may contain concentrations of virtually any solute, including bioactive agents such as vitamins, hormonal agents, antimetabolites, antimicrobial agents, antifungal agents, local anaesthetics, bronchodilators, beta-adrenergic blockers, antihypertensive agents, antidepressants, anticonvulsants, antihistamines, antimalarial agents, analgesics, antibiotics, immunogens, immunomodulators, antigens, nutrients, proteins, peptides, nucleosides, oligo and polynucleotides, ribonucleic acid (RNA) and deoxyribonucleic acid (DNA) and analogs of RNA and DNA, antineoplastic agents, antihistaminic agents, neuropharmacologic agents including sedatives and hypnotics, steroidal and nonsteroidal antiinflammatory agents, diuretic agents, antiarrhythmic agents and vascular dilating agents, among others, including radiographic contrast agents, nuclear magnetic resonance (NMR) contrast agents and antiviral agents. Additional bioactive agents for use in the present invention include nutrients such as proteins, fatty acids and carbohydrates. In certain preferred embodiments of the present invention, radiocontrast agents, NMR contrast agents, peptides and certain antibiotics, for example, cephalosporins are utilized in the present invention. Exemplary radiocontrast agents for use in the present invention include, for example, iohexol, iopamidol, iotroxic acid, ioxoglate, iotrolan, ioversol, iothalamate, iodimide, iodipamide, iopentol, iodixanol, metrizamide, mixtures thereof and their pharmaceutically acceptable salts. Bioactive agents can be naturally occurring, synthetic, or semi-synthetic antimicrobial agents. Exemplary antimicrobial agents are aminoglycosides such as: gentamicin, amikacin and tobramycin. In preferred compositions of the present invention, the IF gels and/or liposomes contain high concentrations of the bioactive agent. In general, the bioactive agent/lipid weight ratio of the IF gels and liposomes of the present invention are as high as from about 1:10 to about 15:1. Of course, these weight ratios are exemplary only and in certain cases it may be necessary to provide drug and lipid in weight ratios above or below these ratios.

In certain embodiments of the present invention the solute or bioactive agent can also function as the inducer. In such cases, it is not necessary to remove the inducer/bioactive agent as in other embodiments of the present invention.

Suitable bioactive agents for use in the present invention include any agent which exhibits biological activity when administered topically or systemically in the liposomes or gels of the present invention. Numerous bioactive agents may be included with the IF gels of the present invention, preferably for topical or oral delivery. The same agents may be included in the IF liposomes of the present invention for topical administration.

The present invention also relates to a method for producing IF liposomes and gels containing a bioactive agent of varying concentration. In the method of the present invention, sized liposomes, preferably about 0.4 µm (microns) in diameter or less, and more preferably about 0.05 µm (microns) in diameter or less and most preferably about 0.025 µm (microns) in diameter, formed by sonication, extrusion, homogenization or an alternative process, or alternatively, FAT MLVs, are fused in the presence of ethanol or other suitable inducer. Depending upon the interaction of the bioactive agent with the lipids used in the IF gels and liposomes of the present invention, the agent may be added before or after the inducer is added. The addition of inducer results in the IF gel of the present invention. The gel including bioactive agent can be administered to a patient, or alternatively, converted to IF liposomes of the present invention.

To produce IF liposomes of the present invention, the IF gels are exposed to a temperature usually but not necessarily above the transition temperature of the lipid used and, additionally, the inducer may be removed. The temperature required by the methods of the invention is that temperature, for any given mixture of lipid, solute or inducer that produces a change in the material properties of the mixture thereby forming the IF gels and IF liposomes of the invention. The result is a composition comprising IF liposomes containing high concentrations of bioactive agent.

The IF liposomes produced by this method may vary in size generally from about 100 µm (microns) to about 0.025 µm (microns), more preferably about 20 µm to about 0.025 µm, and may contain high concentrations of bioactive agent. These IF liposomes may be further size reduced using any of the techniques available in the art.

In a further aspect of this invention, a second material may be incorporated into interdigitation-fusion vesicles by post-gel incorporation, as described in detail in Example 29, below. The second material may be a second lipid, or may be some other material which would otherwise interfere with the interdigitation fusion process.

### Brief Description of the Drawings

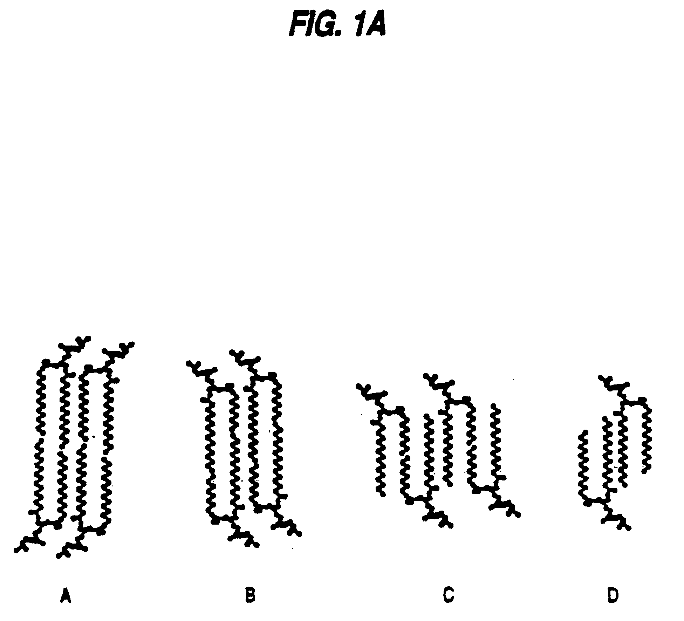

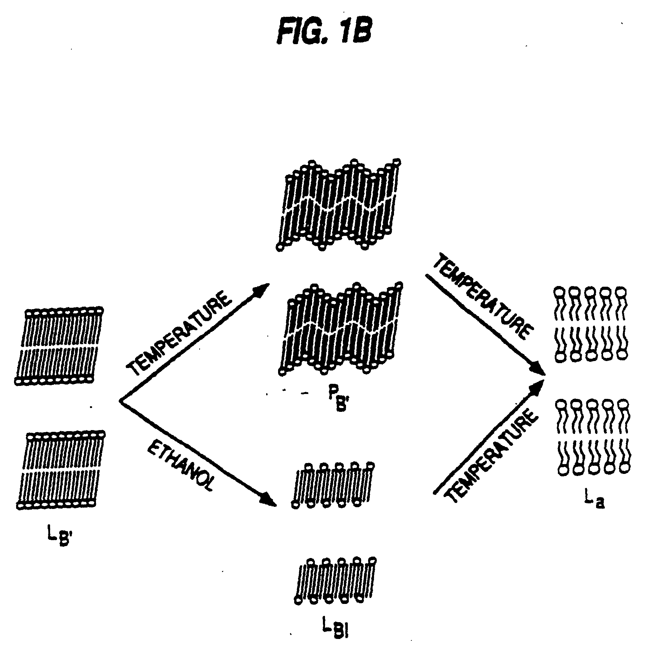

Figure 1A is a schematic representation of the different acyl chain arrangements possible in bilayers. A represents a noninterdigitated bilayer comprising a phospholipid containing a symmetrical, saturated phospholipid C(16):C(16)phosphatidylcholine. B represents a partially interdigitated bilayer comprising an asymmetrical saturated phospholipid C(16):C(10)phosphatidylcholine. c represents a mixed interdigitated bilayer comprising C(16):C(10)phosphatidylcholine. D represents a fully interdigitated bilayer comprising C(16):C(16)phosphatidylcholine in combination with an effective amount of an inducer.
Figure 1B is a schematic representation showing the effect of temperature and an inducer (ethanol) on the interdigitation of a saturated species of phospholipid.

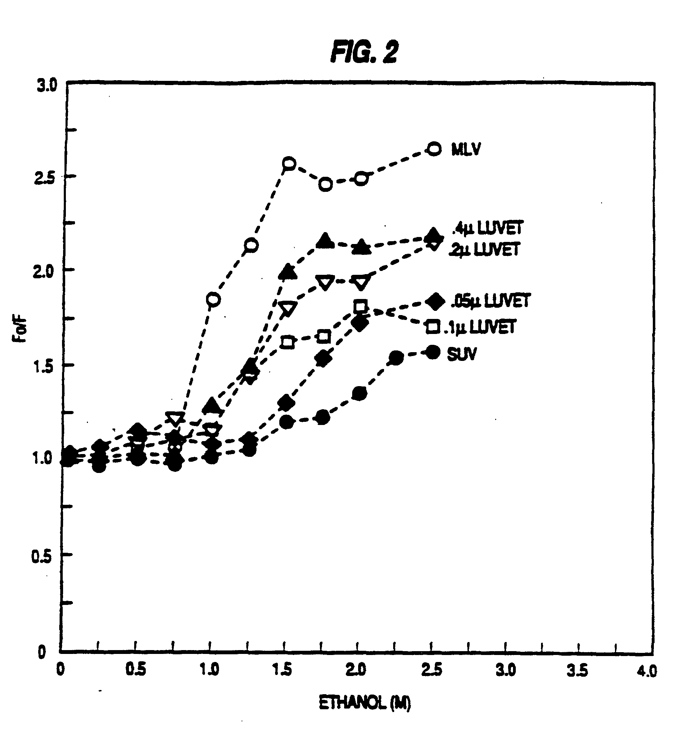

Figure 2 represents interdigitation of Dipalmitoylphosphatidylcholine (DPPC) liposomes as a function of the initial size of the liposomes and the concentration of ethanol. Interdigitation is determined by diphenylhexatriene (DPH) fluorescence intensity. DPH fluoresces maximally when incorporated into the lipid bilayer. Interdigitation results in the reorientation of DPH with a concomitant decrease in fluorescence. Fo = DPH fluorescence in the absence of ethanol. F = DPH fluorescence in the presence of ethanol. Excitation = 351 nm. Emission was detected between 380 and 580 nm and quantitated by weighing.

Figure 3 represents lipid mixing of DPPC liposomes as a function of size and ethanol concentration as judged by resonance energy transfer (RET) between NBD-PE and rhodamine-PE incorporated together in a marker population of liposomes.

Figure 4a graphically represents the ¹⁴C sucrose encapsulation percentage as a function of ethanol concentration. The internal volume of DPPC IF liposomes as a function of increased ethanol concentration is shown in Figure 4b, solid circles representing internal volume determined by ¹⁴C sucrose encapsulation; open circles refer to the CAT 1 EPR measurement. Figure 4c represents the percentage of DPPC recovered as a result of failure of IF liposomes to form at 1.0 M ethanol concentrations, with the result being the SUVs remaining did not successfully centrifuge.

Figure 5 shows internal volumes of IF liposomes formed from various lipids using ¹⁴C sucrose, TEMPONE EPR and CAT 1 EPR methods (solid, diagonal, and shaded bars, respectively).

Figure 6 shows the internal volume of DPPC IF liposomes as a function of initial size of liposomes prior to the addition of ethanol. Internal volumes of these liposomes were calculated by ¹⁴C sucrose encapsulation as well as CAT 1 EPR and TEMPONE EPR methods (solid, diagonal and shaded bars, respectively).

Figures 7a and 7b graphically represent the incorporation of DPPG into DPPG-DPPC IF liposomes. The internal volumes of the IF liposomes are shown in Figure 7a as a function of mole fraction of DPPG (volumes measured by ¹⁴C encapsulation, open circles; volumes measured by the broadening agent TEMPONE EPR technique, closed circles). Figure 7b shows the percent recovery of Pi (closed circles) and ¹⁴C labeled sucrose (open circles) as a function of DPPG.

Figures 8a and 8b graphically represent the internal volume and encapsulation as a function of initial DPPC concentration, wherein Figure 8a shows that the encapsulation percentage of sucrose increases with the initial DPPC lipid concentration. Figure 8b shows the internal volume of the DPPC IF liposomes measured by both the ¹⁴C sucrose method (closed circles) and the EPR method (open circles).

Figure 9a and 9b are Malvern particle size distributions of IF liposomes at (A) 10 mg/ml and (B) 20 mg/ml lipid.

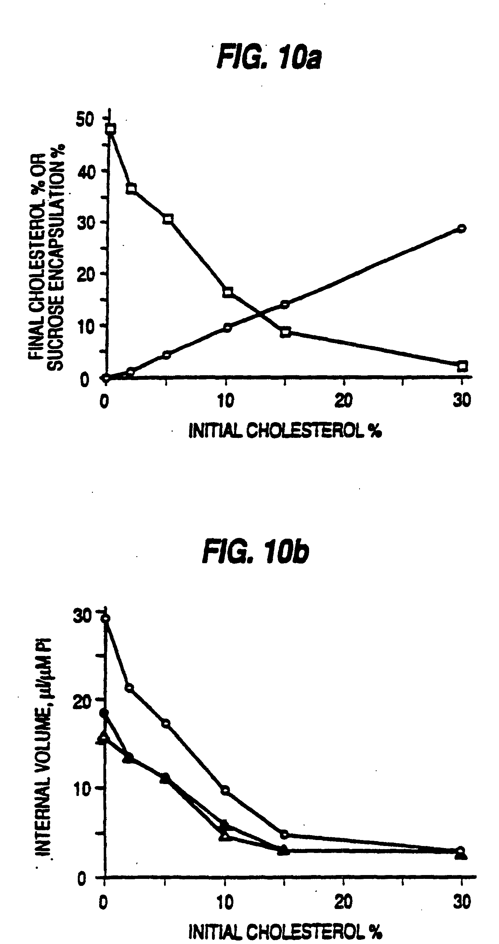

Figures 10a and 10b graphically represent the effects of cholesterol on formation of DPPC IF liposomes. Figure 10a shows the "final" cholesterol concentration of the IF liposomes (open circles) and the final percentage of ¹⁴C sucrose encapsulated (open squares) as a function of initial cholesterol percent of the liposomes prior to addition of ethanol. Figure 10b shows the decrease in internal volume of the DPPC-cholesterol IF liposomes as a function of cholesterol content (¹⁴C sucrose encapsulation, CAT 1 EPR and TEMPONE EPR methods; open circles, open triangles and closed circles respectively).

Figures 11a and 11b graphically represent the effects of dioleoylphosphatidylcholine (DOPC), (unsaturated lipid) on formation of IF liposomes. Figure 11a shows the internal volume of IF liposomes containing varying amounts of DOPC by ¹⁴C sucrose encapsulation and TEMPONE EPR methods (open squares and closed squares, respectively). Figure 11b shows the lipid recovery following the formation of IF liposomes containing varying amounts of DOPC.

Figure 12 is a histogram demonstrating the effect of lipid incubation time above and below the DPPC Tm (5 minutes, 30 minutes, 60 minutes and 120 minutes), and incubation procedure (room temperature "RT", or 50°C), on the resulting internal volume of the IF liposomes. Throughout this specification, the term "RM temp" means 25°C, unless otherwise specified.

Figure 13 is a graph showing the effect of pressure and temperature on formation of PIF vesicles composed of DPPC. Small DPPC liposomes were placed in Teflon® polytetrafluoroethylene sample holders and the indicated pressures were applied for 15 minutes at the indicated temperatures.

Figure 14 is a graph showing the effect of pressure and temperature on formation of PIF vesicles composed of DSPC. Small DSPC liposomes were placed in Teflon® sample holders and the indicated pressures were applied for 15 minutes at the indicated temperatures.

Figure 15 is a graph showing the effect of pressure sterilization on bacteria at 40°C.

Figure 16 is a graph showing the effect of pressure sterilization on bacteria at 50°C.

Figure 17 is a graph showing the effect of pressure sterilization on bacteria at 60°C.

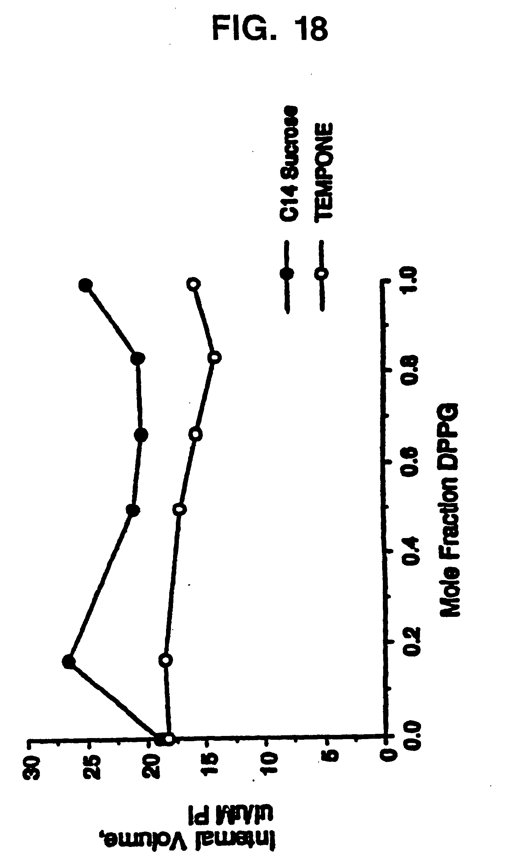

Figure 18 is a graph showing the effect of the mole fraction of DPPG on the internal volume of large DPPC/DPPG interdigitation-fusion vesicles (IFVs).

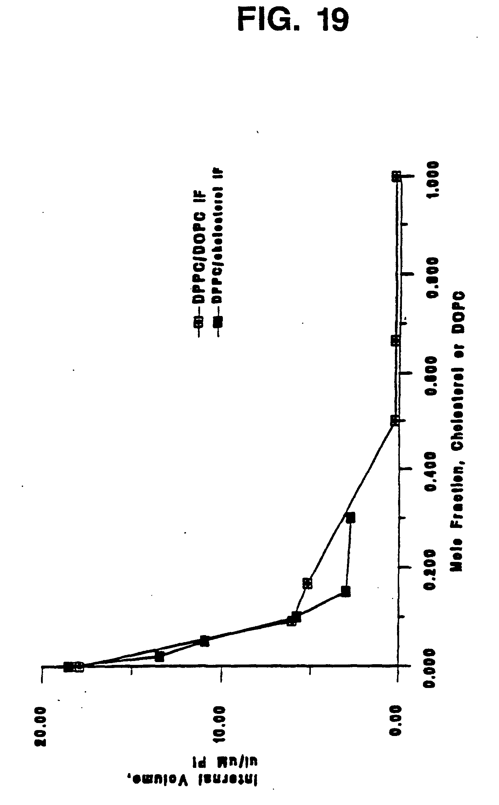

Figure 19 is a graph showing the effect of the addition of DOPC or cholesterol on the internal volume of large DPPC interdigitation-fusion vesicles (IFVs).

Figure 20 is a graph showing the effect of post-gel incorporation of DMPC SUVs on the internal volume of large DPPC IFVs.

Figure 21 is a graph showing the effect of temperature on the membrane fluidity of various DMPC, DPPC, and DPPC/DMPC IFVs.

Figure 22 is a graph showing the effect of post-gel incorporation of cholesterol on the internal volume of large DPPC IFVs.

Figure 23 is a graph showing the effect of post-gel incorporation of DOPC SUVs on the internal volume of large DPPC IFVs.

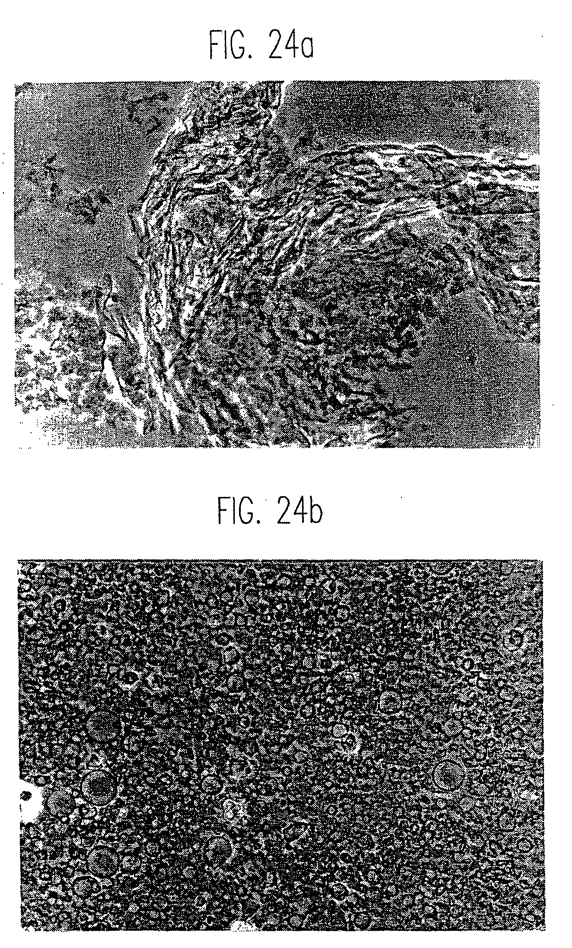

Figure 24 shows phase contrast photomicrographs of the ethanol-induced DPPC interdigitated sheets and DPPC IFVs. A. Photomicrographs (400X) of ethanol-induced DPPC interdigitated sheets formed at 20 mg/ml (3.0M ethanol, 150 mM NaCl, 10 mM Tris-HCl, pH 7.2, room temperature). The sheet suspension was then disrupted by a five-fold dilution with 3.0M ethanol NaCl/Tris buffer. B. Photomicrograph (400X) of 20 mg/ml DPPC IFVs in 3.0M ethanol NaCl/Tris buffer at room temperature. The IFVs were formed from ethanol/DPPC interdigitated sheets by raising the sample temperature above the Tm of the DPPC. The sacle bars in both micrographs indicate 10 µm (microns).

Figure 25 Freeze-fracture electron micrographs of ethanol-induced DPPC interdigitated sheets and DPPC IFVs. A. Ethanol-induced DPPC interdigitated sheets (2.5M ethanol, 150 mM NaCl, 10 mM Tris/HCl, pH 7.2, room temperature). B. DPPC IFVs formed by incubation of the interdigitated sheets above the Tm of DPPC. The scale bars indicate 1 µm (micron).

Figure 26 shows the size distribution of DPPC IFVs. The normalized diameter distributions for three DPPC IFV samples were measured at room temperaturte using a Malvern 3600ER laser diffraction particle sizer. The distributions are based upon particle number and were calculated by that instrument. Average values and standard deviations for the three samples are shown. The number averaged diameter for the samples was 3.54+/-0.12 microns. The IFVs were formed from DPPC SUVs at 20 mg/ml using 4.0M ethanol.

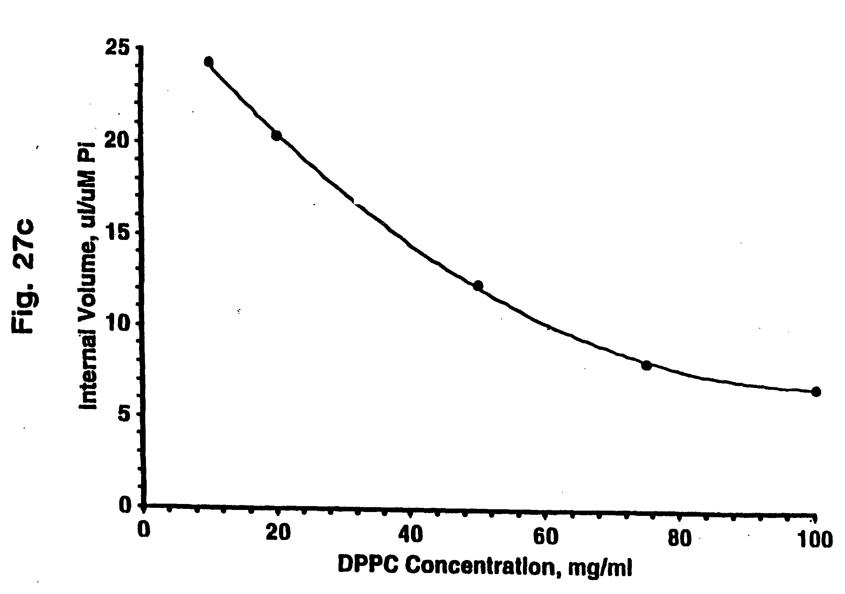

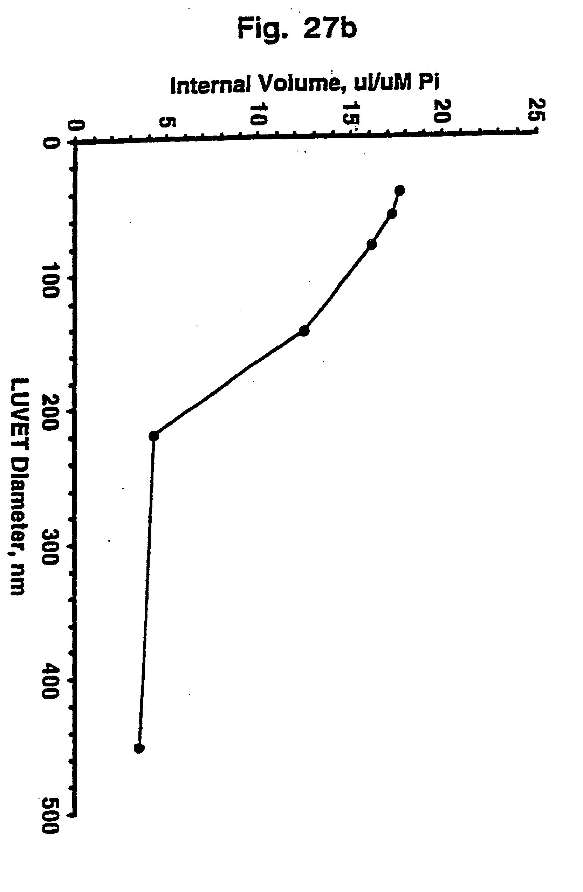

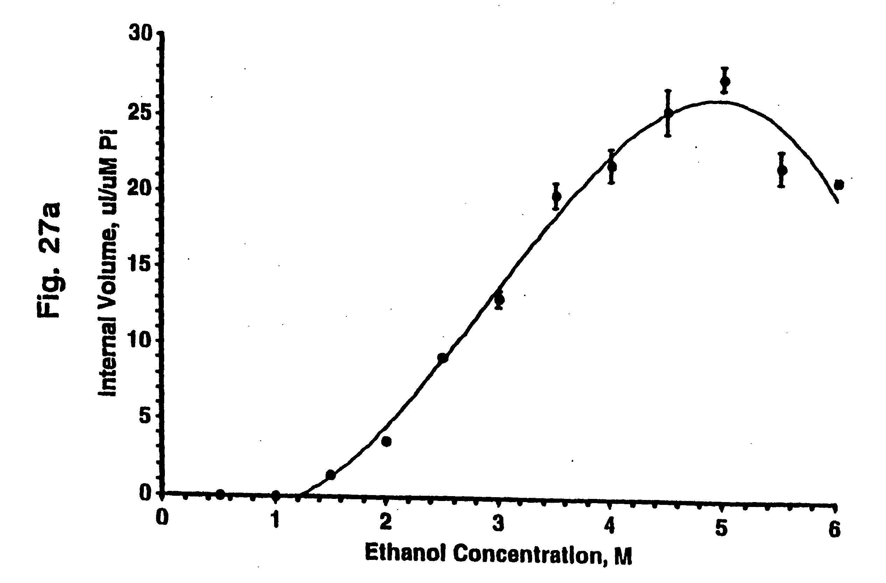

Figure 27 shows the effect of interdigitation-fusion paramters on the internal volume of DPPC IFVs. A. Internal volume as a function of ethanol concentration. For ethanol concentracions greater than or equal to 2.0M, the DPPC IFVs were formed by direct addition of ethanol to DPPC SUVs at 20 mg/ml. At 1.5M or less, ethanol was prediluted before addition to avoid mixing artifacts. Error bars indicate standard deviations. B. Internal volumes of DPPC IFVs formed from DPPC LUVETS of various diameters are shown. The LUVETS were prepared by extruding previously formed MLVs through two polycarbonate filters. Diameters were determined by quasi-electric light scattering. C. Internal volumes of DPPC IFVs formed at different DPPC SUV concentrations. Interdigitation-fusion was induced with 4.0M ethanol.

Figure 28 shows the internal volumes for IFVs made from various saturated acyl chain phosphatidylcholines. Average internal volumes are shown for DMPC, DPPC, DHPC, DSPC and DAPC IFVs which were prepared from SUVs at 20 mg/ml using 4.0M ethanol to induce interdigitation-fusion. Error bars indicate the stndard deviations.

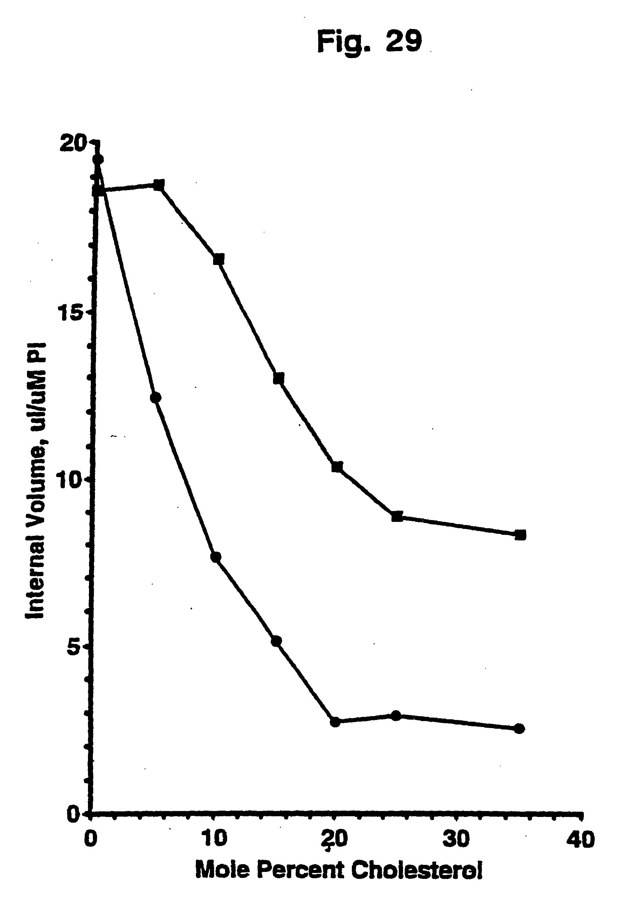

Figure 29 Incorporation of cholesterol into DPPC IFVS is shown. Final internal volumes of DPPC/Cholesterol IFVs are compared for two methods of cholesterol incorporation into IFVs. DPPC. Filled circles indicate DPPC/Chol IFVs formed directly from DPPC/Chol SUVs of varying mole percent cholesterol. With this method, the internal volumes of the product IFVs rapidly decreased with increased cholesterol content. Filled squares indicate internal volumes of DPPC/Chol IFVs formed when cholesterol was added in the form of 1:1 DPPC/Chol SUVs in 4.0M ethanol after ethanol-induced DPPC interdigitated sheets were formed. Significantly higher internal volumes were produced at each mole percent cholesterol used. Differential scanning calorimetry was used to demonstrate that cholesterol was incorporated directly into bilayers. At 35 mole percent cholesterol, the DPPC gel-to-liquid crystalline phase transition was eliminated in DPPC/Cholesterol IFVs formed by either method.

### Detailed Description of the Invention

For purposes of clarity, throughout the discussion of the present invention, the following definitions will be used:
"Interdigitation" and "interdigitated" are used throughout the specification to describe a lipid bilayer in which the acyl chain region of one lipid in a bilayer interpenetrates into the other layer of the lipid bilayer. The term interdigitation shall include full interdigitation, mixed interdigitation and partial interdigitation. Full interdigitated liposomes include interdigitated liposomes in which the acyl chains of the lipid interpenetrate fully or partially across the width of the lipid bilayer as in Figure 1A(D). Mixed interdigitated liposomes include interdigitated liposomes in which certain acyl chains of unsymmetrical phospholipids, generally the longer acyl chains, extend completely across the bilayer span, whereas the shorter chains meet end to end in the bilayer midplane as in Figure 1A(C). Another example of mixed interdigitation liposomes includes liposomes in which regions of the liposome are either fully or partially interdigitated and may co-exist with regions that are not interdigitated. Partially interdigitated liposomes include interdigitated liposomes in which the acyl chains of unsymmetrical phospholipids pair such that the longer acyl chain of one bilayer pairs with the shorter acyl chain of the other bilayer as in Figure 1A(B).
"Inducer" is used throughout the specification to describe molecules, including amphipathic molecules of limited size which localize at the lipid bilayer aqueous phase interface region of a liposome and produce an interdigitation-fusion gel which may also be a liquid, of the present invention. The term also contemplates lipids and/or solutes such as bioactive agents that may act as "self-inducers". Hydrostatic pressure may also be an inducer.
"Pressure induced fusion" ("PIF") is interdigitation induced by a sufficient application of hydrostatic pressure applied to sized liposomes to form an interdigitation fusion gel. Liposomes formed from this gel are referred to herein as PIF liposomes, or simply PIFs.
"Interdigitation-fusion gel" (IF gel) is used throughout the specification to describe the product that results when an inducer is combined in sufficient quantity to fuse sized liposomes. The resulting sheets of lipid are fused gels for purposes of the present invention and may include products of varying viscosity including liquids, gels and in certain cases, even very viscous products approaching the solid state.
"Interdigitation-fusion liposome" (IF liposome) is used throughout the specification to describe the liposome that results from IF gels which are generally but are not necessarily raised above the lipid transition temperature ("Tm"), but in any case are incubated at a temperature to produce IF liposomes. The inducer may additionally but not necessarily be removed from the interdigitation-fusion gel. In certain embodiments in which the liposome contains self-inducing lipid or the solute is an inducer, the inducer is not removed. The IF liposomes may contain large concentrations of solute such as bioactive agents in bioactive agent:lipid ratios of about 1:10 to 15:1.
"Solute" is used throughout the specification to describe any chemical, including buffers and solvents that may be entrapped by the IF gels and liposomes of the present invention. Solutes may include buffers, salts, toxins, microbes and bacteria, pesticides, insecticides, herbicides, fungicides, emulsifying agents, cosmetics, unicellular organisms and a large number of chemical agents, especially including bioactive agents.
"Bioactive agent" is used throughout the specification to describe any agent such as chemical agents which exhibits. biological activity when administered to living organisms, including plants, animals such as mammals, and especially including humans. Bioactive agents include drugs and nutrients, among others as described hereinabove and following.
"Saturated lipid" is used throughout the specification to describe a lipid that may be used to produce the interdigitation-fusion gels and liposomes of the present invention. The term saturated lipid includes, but is not limited to, lipids having symmetrical and/or asymmetrical acyl side chains which are saturated, i.e., contain no double bonds, lipids having unsaturated side chains in which the unsaturated carbon-carbon double bonds are oriented in the trans configuration and certain lipids having unsaturated side chains in which the unsaturated carbon-carbon double bonds are oriented in the cis configuration, or mixed fatty acid lipids such as for example SOPC and POPC.
"Interdigitation-fused lipid containing composition" is used to describe the IF gels and liposomes of the present invention.

The present invention relates to lipid containing compositions comprising a sized liposome, preferably about 0.4 µm (microns) or less to about 0.05 µm (microns) or less and more preferably about 0.025 µm (microns) in diameter or less, and an amount of an inducer effective to fuse the liposomes in combination with a solute. The initial liposomes may alternatively be FAT MLVs. In certain embodiments the solute is a bioactive agent. The compositions of the present invention may advantageously include bioactive agent at high concentrations, for example at bioactive agent:lipid ratios of about 1:10 to 15:1.

In the present invention, the sized liposomes which give rise to IF gels and liposomes of the present invention are preferably formed from zwitterionic, cationic, and anionic lipids and phospholipids comprising fatty acyl chains, having 12 to 35 carbon atoms, also including therein saturated (disaturated and partially saturated) and unsaturated and polar or apolar lipids and phospholipids.

For example, the saturated lipids of the invention include but are not limited to for example, dimyristoylphosphatidylcholine, distearoylphosphatidylcholine, dipalmitoylphosphatidylcholine, dimyristoylphosphatidylserine, dipalmitoylphosphatidylserine, distearoylphosphatidylserine, dimyristoylphosphatidylethanolamine, dipalmitoylphosphatidylethanolamine, distearoylphosphatidylethanolamine, dimyristoylphosphatidic acid, distearoylphosphatidic acid, dipalmitoylphosphatidic acid, dimyristoylphosphatidylinositol, distearoylphosphatidylinositol, dipalmitoylphosphatidylinositol, hydrogenated soy phosphatidylcholine, hydrogenated soy lecithin, dipalmitoylphosphatidylglycerol, di-0-hexadecylphosphatidylcholine, dipalmitoylphosphatidylglycerol, distearoylphosphatidylglycerol, dimyristoylphosphatidylglycerol, among others.

Other saturated lipids include but are not limited to the saturated lipids having symmetrical and/or asymmetrical acyl side chains which are saturated, i.e., contain no double bonds, lipids having unsaturated side chains in which the unsaturated carbon-carbon double bonds are oriented in the trans configuration and certain lipids having unsaturated side chains in which the unsaturated carbon-carbon double bonds are oriented in the cis configuration, or mixed fatty acid lipids such as for example SOPC and POPC.

Other lipids for inclusion with the saturated symmetrical lipid include other liposome forming lipids including, for example synthetic or natural phospholipids including mixed chain compositions, for example, phosphatidylcholines (PC), phosphatidylethanolamines (PE), phosphatidylserines (PS), phosphatidylglycerols (PG), phosphatidic acids (PA), phosphatidylinositols (PI), sphingomyelins (SPM) and cardiolipins, among others, either alone or in combination.

In addition to the above lipids, additional lipids including various lysolipids, for example, n-octadecyl-2-methylphosphatidylcholine, n-octadecylphosphatidylcholine, 1-laurylpropanediol-3-phosphocholine, erythro-N-lignoceroylsphingophosphatidylcholine, cholesterol, and water soluble derivatives thereof such as for example cholesterol hemisuccinate and alpha tocopherols and water soluble derivatives thereof such as tocopherol hemisuccinate, and gangliosides, glycolipids, and glycosphingolipids which may also be included in compositions of the present invention. One of ordinary skill in the art will recognize that the amount and type of lipid which may be included in compositions of the present invention may be varied within the teachings of the present application to produce the compositions according to the present invention.

In the compositions of the present invention, the sized liposomes containing significant quantities of at least one saturated lipid are interdigitated-fused with addition of an inducer. The sized liposomes of the present invention generally containing a saturated phospholipid will undergo full, partial or mixed interdigitation in combination with an effective amount of the interdigitation inducer. While not being limited by way of theory, it is believed that the inducer may function to displace some of the headgroup-associated water molecules and in general, causes an increase in the headgroup surface area. It is preferred that the lipids chosen should undergo full interdigitation in the presence of the inducer; however, it is to be recognized that lipids which provide less than complete interdigitation, i.e., either mixed or partial interdigitation are also contemplated and are within the scope of the present invention. Exemplary interdigitation inducers for use in the present invention include, for example, short chain alcohols including methanol, ethanol, propanol, isopropanol and n-butanol, polyols such as glycerol and ethylene glycol, anaesthetics such as chlorpromazine, tetracaine, phenylethanol, benzyl alcohol and phenylbutanol, among others, buffers such as Tris and chaotropic salts such as thiocyanate SCN-, as well as others referred to hereinabove.

In certain cases, the saturated lipid used to form the IF gels and liposomes of the present invention are self-inducers, i.e., these lipids will interdigitate and form IF gels and liposomes by mixing the lipid in the presence of solute at varying temperatures without the need to add a chemical inducer (for example, DHPC). In addition, in certain cases extremely high pressure may be used to produce interdigitation without the need to include an inducer.

In accordance with one aspect of the present invention, interdigitation may be induced by the application of hydrostatic pressure to a population of sized liposomes. The period of time and level at which the pressure is applied to the liposomes must be effective to cause the interdigitation fusion to occur. Although no specific minimum pressure is required, a preferred pressure level for liposomes comprised of saturated lipids is at least about 1378,8 x 10⁵ Pa (20 000 psi) preferably at least about 2 757,6 x 10⁵ Pa (40000 psi). The pressure should be applied for a period of time sufficient to cause interdigitation fusion, preferably about one minute to about one hour.

Good results were obtained using liposomes comprised of either dipalmitoylphosphatidylcholine (DPPC) or distearoylphosphatidylcholine (DSPC). In one example, small unilamellar vesicles (SUVs) comprising DPPC or DSPC were fused to form larger liposomes by the application of pressure of at least 1378,8 x 10⁵ Pa (20000 psi), and preferably at least 2757,6 x 10⁵ Pa (40000 psi) for a period of about 15 minutes. During these tests, at least a partial fusion of the small liposomes was noted at the first observation after 5 minutes. This process is discussed in more detail in Example 27, below. Although DPPC and DSPC were successfully interdigitated by pressure induced fusion (PIF), the PIF process was found not to induce interdigitation on small liposomes of palmitoyloleoylphosphatidylcholine (POPC), which has asymmetrical acyl side chains.

As a further aspect of this embodiment of the present invention, high hydrostatic pressures may be used co kill bacteria and to sterilize liposomal preparations. Thus, hydrostatic pressure can be used not only to induce interdigitation fusion, and but also to sterilize liposome preparations. This sterilizing process can be applied as part of an interdigitation fusion process, or can be used to sterilize liposomes formed by other processes, such as, for example, the methods described in U.S. Patent Nos. 4,522,803, 4,588,578 and 4,975,282, discussed above.

Hydrostatic pressures have long been known to have effects on cellular processes. Currently, high pressure and moderate temperature is used for the pasteurization and sterilization of certain food products. Such methods generally employ the application of pressures as high as 5170,5 x 10⁵ Pa (75 000 psi) and moderate temperatures. Bacteria, yeasts and viruses may all be inactivated by the application of high pressure. The inactivation process, given a fixed pressure, is known to vary as a function of temperature, chemical composition of the medium, and time.

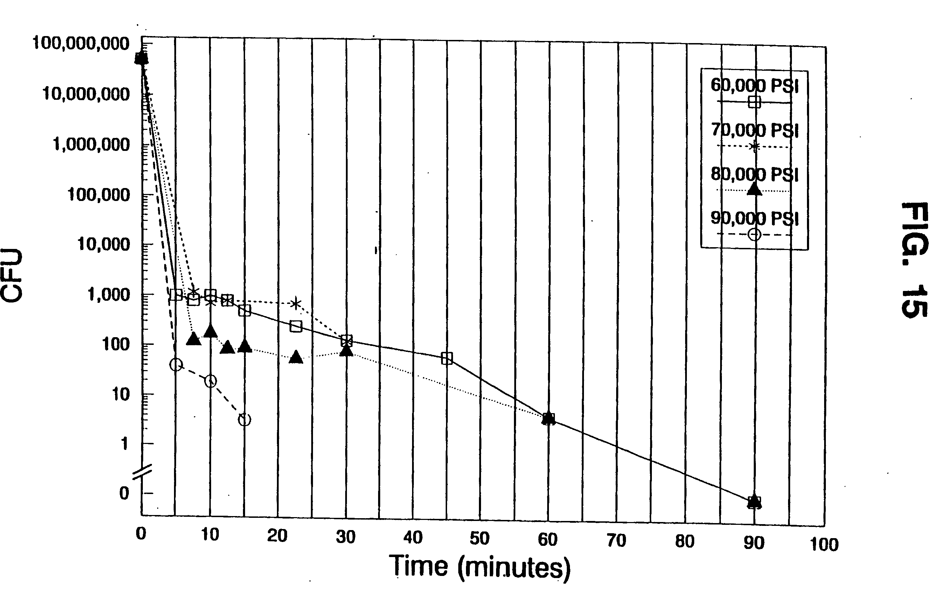

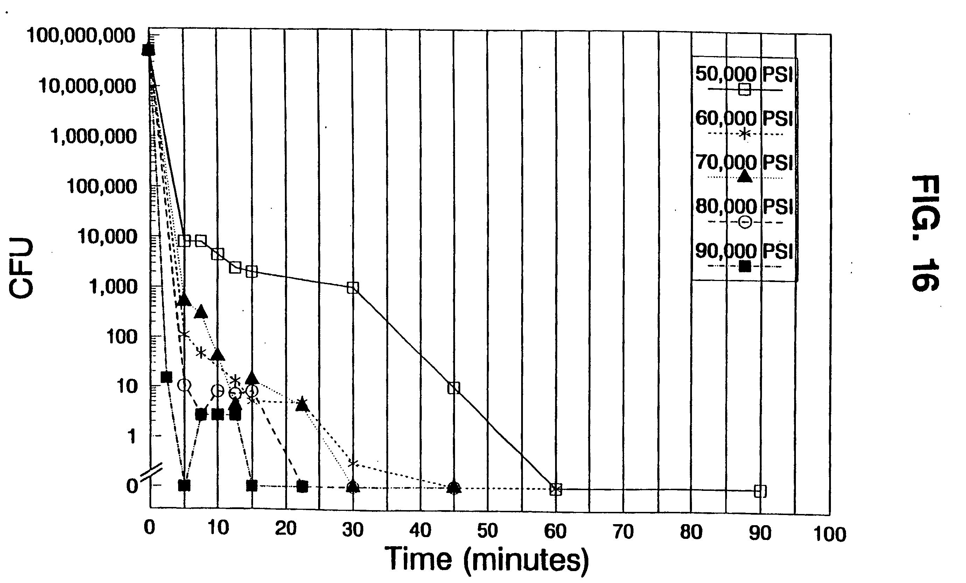

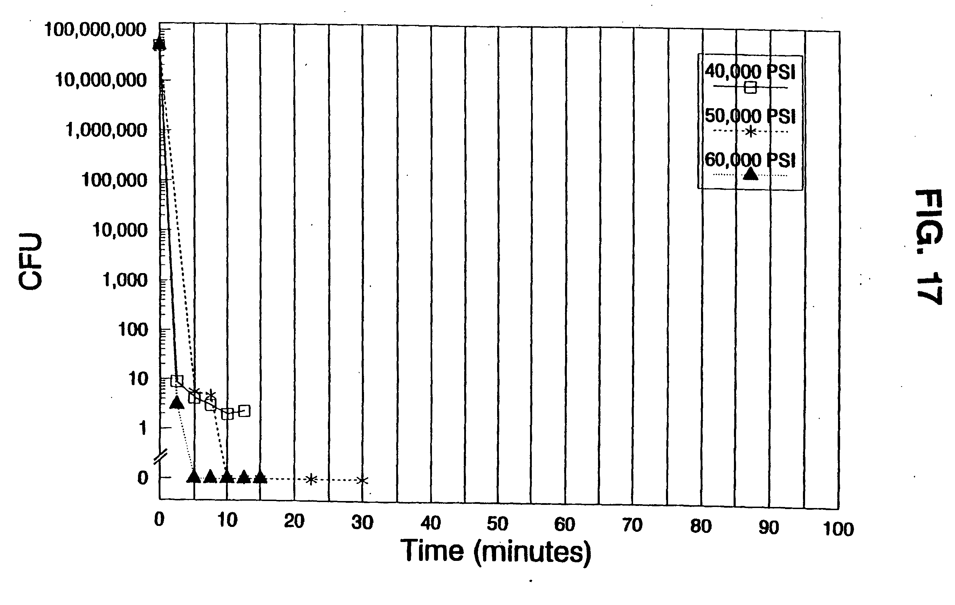

In accordance with the present invention, high hydrostatic pressures and moderate temperatures are used to kill microbes, including bacteria such as *Bacillus subtilis* in Example 28 below, and thereby to sterilize liposomal preparations. *Bacillus subtilis* was chosen for use in this example because it is considered one of the most difficult microbes to kill in sterilization processes. In Example 23, the rate of inactivation for several temperatures at a number of pressures is described. As can be seen from the results presented in Figures 15, 16 and 17, the rate of sterilization is a function of the temperature and pressure at which the sterilization is performed.

In general, in performing the sterilization process of the present invention on a selected liposomal composition in the shortest time, one would determine a suitable temperature and pressure effective for composition. For the most rapid sterilization, one would use a combination of the highest temperature and pressure which can be tolerated by the liposomal composition. The data presented in Figures 15, 16 and 17 for Example 28 provide an example of the relationship between temperature and pressure for such sterilizing processes.

A pressure of about 689,4 x 10⁵ Pa (10000 psi) is considered to be a minimum level for effecting sterilization in accordance with the present invention. Preferably, a minimum of about 2757,6 x 10⁵ Pa (40 000 psi) pressure is used to carry out the sterilization more rapidly, the actual time required being dependent on the temperature under which the process is performed. The pressure should be kept below a level which can have a deleterious effect on the particular liposome composition being sterilized, either by damaging the liposome structures or by affecting any of the components of the composition. In like manner the maximum temperature should also be below that which causes deleterious effects to the liposome composition, such as structural or chemical changes to the liposomes or to any of the components of the composition.

In general, the compositions of the present invention include an amount of an inducer effective for fusing the sized liposomes. The amount and type of inducer utilized to produce liposome fusion will vary as a function of the type of liposome utilized. In general, however, the amount of inducer used comprises about 1.0% to about 50% of the total weight of solution which includes a combination of the sized liposomes, inducer and solute. One of ordinary skill in the art will recognize to readily vary the concentration of the inducer within the teachings of the art and the present application to produce interdigitation gels and liposomes of the present invention.

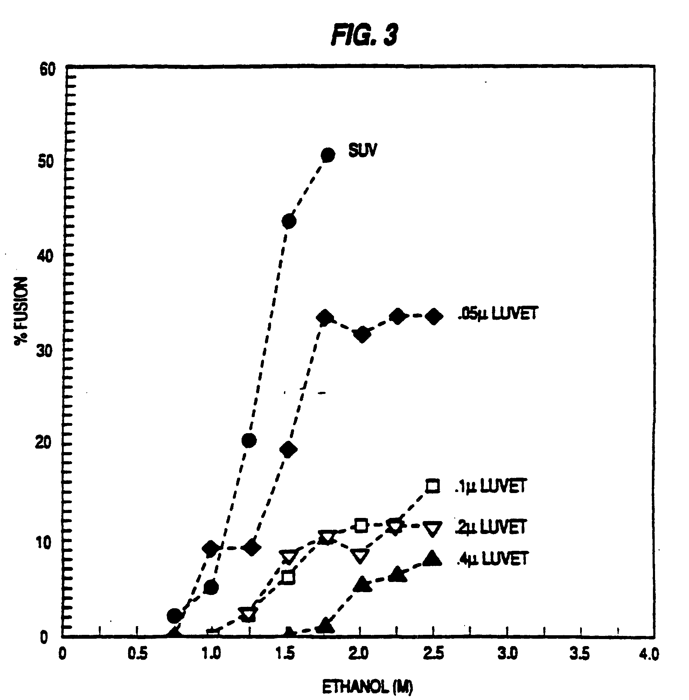

While not being limited by way of theory, it is believed that sized liposomes fuse into lipid sheets (gels) at certain concentrations of inducer in order to relieve bilayer strain imposed by a small radius of curvature (See, for example, Figure 3). The resulting interdigitation-fusion gel that is produced may capture a high concentration of solute. This includes encapsulating substances which otherwise cannot be entrapped in high solute lipid ratios in liposomes. According to the method of the present invention, when the IF gels are exposed to temperatures usually but not necessarily above their L beta I-L alpha transition temperature ("Tm"), but in any case at a temperature which changes the material properties of the mixture such that IF liposomes are formed, and the inducer is preferably (but not necessarily) removed, liposomes of high captured volume result. These liposomes may vary in size as a function of the solute, liposome and inducer utilized, but generally, will range in size from about 100 µm and more preferably about 20 µm (microns), to about 0.025 µm (microns).

While not being limited by way of theory, it is believed that interdigitation, which renders the lipid bilayer less susceptible to perturbation during liposome formation, can be utilized to capture substances which normally interact with membranes and are difficult to entrap. For example, interdigitation-fusion liposomes of the present invention have been used to entrap high concentrations of aminoglycosides which are very difficult to entrap in high concentrations because of their tendency to interact with membranes. IF liposomes have been shown to entrap gentamicin at a drug/lipid ratio of about 1:2 (w:w) whereas typically modified small plurilamellar liposomes (SPLVs) entrap gentamicin at a drug/lipid ratio of about 1:10 (w:w).

The production of interdigitation-fusion gels and liposomes of the present invention involves the initial formation of sized liposomes about 0.4 µm (microns) in diameter or less, more preferably about 0.05 µm (microns) or less and most preferably no greater than about 0.025 µm (microns).
Alternatively, FAT MLVs can be used, and in some cases, larger liposomes can be used. Any of the methods available in the art for producing sized liposomes may be utilized including the methods described in greater detail hereinbelow. Typically, liposomes can initially be prepared by vacuum drying a solution of lipid in organic solvent, for example, chloroform, to a thin film in a round bottom or other suitable flask or vessel, followed by hydration of the lipid film with an aqueous solvent such as for example, aqueous buffer or saline solution. Alternatively, liposomes can be formed from admixture of dry lipid powder and aqueous solvent, preferably for example saline solution or aqueous buffer.

The liposomes are then sized according to any methods known in the art such as sonication, extrusion or homogenization, and further described hereinbelow. After the formation of sized liposomes, the solute, preferably a bioactive agent that is to be encapsulated, is generally mixed in the aqueous solvent. Two approaches are generally used to entrap solute depending upon whether or not the solute interacts with liposomes. In the case where the solute does not interact with the liposomes, the solute may be mixed in with the aqueous or aqueous/buffer solvent after formation of the sized liposomes which are to undergo interdigitation. In the case of solute that interacts with the liposomes, the solute is generally mixed in with the aqueous solvent after the formation of interdigitation-fusion gels.

Of course, one of ordinary skill in the art will recognize that the order in which the individual components of the IF gels and liposomes of the present invention are added may vary and is dependent on the type of solute to be entrapped and the type of saturated lipid utilized.

The final concentration of the lipid used to encapsulate solute of the present invention will vary as a function of the concentration and type of the solute desired as well as the type of lipid used, but in general, the weight ratio of drug to lipid in the aqueous solvent will range from about 50:1 to about 1:100 with the final concentration of lipid falling within the range of about 5 to 100 mM. The final weight ratio of drug to lipid in the interdigitation-fusion gels and liposomes of the present invention ranges from about 1:10 to about 15:1.

After the sized liposomes are formed, inducer is added to the aqueous solvent. The amount of inducer added generally ranges from about 1.0% by weight (of the combined weight of lipid, solute and inducer) up to about 50 percent by weight. Where ethanol is used as the inducer, the amount of ethanol included is generally about 5% by weight (1.0 M) to about 20% by weight (4.0 M) and in the case of glycerol the amount of inducer utilized may be as much as about 90-100% by weight. The amount of other inducers to be included will vary. In the case of ethanol the final ethanol concentration falls within the range of about 0.50 to about 10.0 Molar and preferably is within the range of about 1.75 to about 4.0 Molar.

The presence of inducer in an effective amount will cause the sized liposomes to fuse, resulting in fused sheets of lipid. The IF gel produced by this method may be used topically or for oral administration, for example, as formulations encapsulated in soft gelatin or other oral dosage forms. Alternatively, the gel may be further modified to produce the IF liposomes of the present invention.

To produce IF liposomes of the present invention, the mixture is incubated at a temperature for a period of time sufficient to form a gel. Typically this period ranges from about 1 minute to about 1 hour. Thereafter, the temperature is generally but not necessarily raised above the Tm of the lipid for a period of about 1 minute to about 1.0 hour. The incubation temperature required may be the Tm of the mixture but is that temperature for any given mixture of lipid, solute or inducer which produces a change in the material properties of the mixture, thereby producing the IF liposomes of the invention. While maintaining this incubation temperature, the inducer may be removed by evaporation (especially in the case of alcohol inducers), positive pressure nitrogen (e.g., N₂ sparge consisting generally of bubbling N₂ through the mixture) or by dilution. This produces IF liposomes varying in size generally between about 0.025 and about 100 µm, more preferably about .025 to about 20 microns. Unencapsulated drug may be removed from the solvent, if desired. The IF liposomes produced by the above method may be further size reduced to produce liposomes varying or homogeneous in size.

In addition to extrusion, initial liposomes (prior to addition of inducer) for the IF gel or liposome method, and resulting IF liposomes may be size reduced by sonication or homogenization. Sonication employs sonic energy to disrupt or shear the larger liposomes which will spontaneously reform into small liposomes. See, for example, Chapman, et al., BBA, 163, 255 (1968). Sonication is achieved by immersing a glass tube containing the liposome suspension into the sonic epicenter produced in a bathtype sonicator. Alternatively, a probe type sonicator may be used in which the sonic energy is generated by vibration of a titanium probe which is in direct contact with the liposome suspension.

With homogenization the shear forces which break down larger liposomes into smaller ones are generated by, for example, rotorstator type devices such as the Polytron (Brinkman Instruments Co., Westbury, New York, USA), a stator-stator type device such as the Microfluidizer (Microfluidics Corp., Newton, MA, USA), or any number of other such devices which are commonly used to disrupt cells. Due to the fact that all of the above methods involve disruption of the IF liposomes, entrapped solute will be lost when IF liposomes are subjected to any of these procedures. The loss may be minimized however, if the unentrapped solute is not removed from the liposome suspension before size reduction of the liposomes.

A number of other techniques may be used for producing sized liposomes which are to undergo interdigitation-fusion, and for producing sized IF liposomes after the process is complete. These methods include reverse-phase evaporation. infusion procedures, homogenization, sonication, microfluidization and detergent dilution or a combination of these methods. A review of certain of these and other methods for producing liposomes can be found in the text Liposomes, Marc J. Ostro, ed., Marcel Dekker, Inc-, New York, 1983, Chapter 1.
Sized liposomes may also be produced by an extrusion process.

In the extrusion process, to produce sized liposomes, the liposomes are passed through filters having pore sizes generally ranging from about 30 nm to about 1 µm (micron) to produce liposomes ranging in size from about 30 nm to about 1 µm (micron) in diameter. Preferably, the pore size of the filters through which the liposomes may be extruded ranges from about 100 nm to about 1 µm (micron). The filters are generally made of polycarbonate, but the filters may be made of any durable material which does not interact with the liposomes and which is sufficiently strong to allow extrusion under sufficient pressure. Preferred filters include "straight through" filters because they generally can withstand the higher pressure of the preferred extrusion processes of the present invention. "Tortuous path" filters may also be used. In the preferred embodiments of the present invention, pre-IF fusion liposomes are extruded through 50 to 100 nm polycarbonate filters to produce liposomes having a diameter of about 50 to 100 nm.

Any extrusion process available in the art may be used to produce sized liposomes which will undergo interdigitation-fusion. The extrusion process may be performed sequentially or once under high pressure. Particularly preferred extrusion processes for use in the present invention include those disclosed in Cullis, et al., PCT Application PCT/US85/01151, Publication Number WO 86/00238 entitled "Extrusion Techniques for Producing Liposomes", published January 16, 1986.

Other methods for sizing the liposomes of the invention either before or after fusion are filtration methods employing asymmetric filters such as for example, Anotec^{R} filters according to commonly-assigned copending U.S. Patent application entitled "Liposome Extrusion Process", U.S. Serial No. 593,200, filed October 5, 1990, which involves extruding liposomes through a branched pore type aluminum oxide porous filter.

Alternatively, the liposomes can be sized using a homogenization or milling procedure such as a colloid mill for example the Gifford Wood colloid mill. The liposomes may be passed one or more times through the mill until the appropriate size and homogeneity is achieved, analyzed for size distribution using either the Nicomp Particle sizer or the Malvern Particle sizer. The liposomes, alternatively, may be passed through a Microfluidizer device, discussed hereinabove, which likewise homogenizes the liposomes.

If desired, the resulting liposomes can be separated into populations using any methods known in the art for so separating: such a process is for example tangential flow filtration. This process as used for the separation of liposomes according to size is disclosed in commonly assigned and copending U.S. Patent application entitled "Method for Size Separation of Particles", Serial No. 225,327, filed July 28, 1988.

In this procedure, a heterogeneously sized population of liposomes is passed through one or more tangential flow filters thereby resulting in a size distribution with an upper and/or lower size limit. For example, when two filters of differing sizes are employed, for example, a first filter of 5 µm pore size, liposomes less than 5.0 µm pass through the filter and into the filtrate, which is then passed through a second filter of smaller pore size, for example, 2.0 µm pore size. In this case, the retentate contains liposomes of a homogeneous size distribution having discrete size limits of 5.0 and 2.0 µm. Filters of alternative pore size may be employed to result in discrete populations having upper and lower size limits.

The liposomes which undergo interdigitation-fusion in the presence of an inducer preferably are about 0.4 µm (microns) in diameter or less, more preferably about 0.05 µm (microns) or less, and most preferably are about 0.025 µm (microns) or less in diameter. Alternatively, the initial sized liposomes of the invention may be FAT MLVs. The IF liposomes which are produced by the general method of the present invention generally range in size from about 100 µm but more preferably about 20 µm (microns) to about 0.025 µm (microns), and generally in the range of about 2 to 20 µm (microns). These resulting IF liposomes may be further size reduced to produce liposomes of varying sizes by sonication, homogenization and extrusion techniques described hereinabove. It should be noted, however, that although these IF liposomes of the present invention may be down sized, the down sizing often results in the loss of bioactive agent from the liposomes. Thus, IF liposomes which undergo further down-sizing, for example, by the previously discussed extrusion process or other processes such as sonication and homogenization to vary the size of the liposomes produced, may encapsulate diminished concentrations of bioactive agents.

Bioactive agents for use in the present invention may include vitamins, hormonal agents, anti-metabolites, antimicrobial agents, antifungal agents, antibiotics, proteins, peptides, ribo and deoxyribonucleic acids, nucleotides, nucleosides, oligonucleotides, antihistaminic agents, neuropharmacologic agents including sedatives and hypnotics, steroidal and nonsteroidal antiinflammatory agents, diuretic agents, antihypertensive agents, antiarrhythmic agents, immunogens, immunomodulators, contraceptive agents, radiographic contrast agents, NMR contrast agents, antiviral agents and vascular dilating agents, among others. In certain preferred embodiments of the present invention, radiocontrast agents, NMR contrast agents, peptides and naturally occurring, synthetic and semi-synthetic antimicrobial agents, for example, cephalosporins and aminoglycosides are utilized in the present invention. Exemplary radiocontrast agents for use in the present invention include, for example, iohexol, iopamidol, ioxoglate, iotrolan, ioversol, iothalamate, iodimide, iodipamide, iopromide, iopentol, iodixanol, metrizamide, mixtures thereof and their pharmaceutically acceptable salts. Exemplary aminoglycosides include gentamicin, tobramycin and amikacin.

Suitable biological agents for use in the present invention include any agent which exhibits favorable biological activity when administered topically or systemically and is stable to the compositions of the present invention. Agents which may be topically administered for their affect on the skin include salicylic acid, resorcinol, phenol, retinoic acid, and their equivalents. Other agents for use in the present invention include certain desensitizing agents, for example antigens and vaccines, vitamins, nutrients, such as amino acids, essential fats and minerals, retinoids, anti-neoplastic and anti-tumor agents, including certain alkylating agents, among others.

Additional bioactive agents for use in the present invention include the benzodiazepines, antipyretic agents, antispasmodics, antipruritic agents, sympathomimetics, decongestants, tranquilizers, antispasmodics, cardioactives, other cardiac agents, anti-emetics, sedatives and hypnotics, steroidal agents, progestational agents, local anesthetics and antibiotics. Other antimicrobial agents may also be used in the present invention including antifungal agents, among others.

The above-listed group of bioactive agents, among other agents, including their pharmaceutically acceptable salts, are contemplated for use in the present invention. Determination of compatibilities of the above listed agents with and the amounts to be utilized in compositions of the present invention are within the ordinary skill in the formulation art. The stability and applicability of individual pharmaceutical agents are well within the ordinary skill of practitioner in this art.

It will be appreciated that the actual preferred amounts of bioactive agent utilized in a specific case may vary according to the severity of a pharmacological or disease condition and the expected pharmacokinetics of bioactive agent in the individual patient. Dosages for a given host can be determined using conventional considerations, e.g., by customary comparison of the differential activities of the subject bioactive agent by means of an appropriate, conventional pharmacological protocol.

The IF liposomes and gels of the present invention may be administered to any animal including mammals, such as humans. For administration to humans in the treatment of afflictions, the prescribing physician will ultimately determine the appropriate dose for a given human subject, and this can be expected to vary according to the age, weight, and response of the individual as well as the nature and severity of the patient's symptoms. The present invention provides a readily available method to allow wide variations in liposomal drug concentrations.

The mode of administration of compositions of the present invention may determine the sites in the organism to which the compositions will be delivered. For instance, delivery to a specific site of infection may be most easily accomplished by topical application (if the infection is external, e.g., on areas such as the eyes, skin, in the ears or on afflictions such as wound or burns) or by absorption through epithelial or mucocutaneous linings (e.g., nasal, oral, vaginal, rectal, gastrointestinal, mucosa, etc.). Such topical application may be in the form of creams or ointments. The interdigitation-fusion gels of the present invention are preferably used topically. However, the IF gels of the present invention may be used orally in formulations in which the lipid, upon contacting the fluids of the mouth or gastrointestinal tract forms a liposome in situ.

The IF liposomes containing bioactive agent may be administered alone but will generally be administered in admixture with a pharmaceutical carrier selected with regard to the intended route of administration and standard pharmaceutical practice, thereby forming pharmaceutical compositions. The IF liposomes of the present invention may be injected parenterally, for example, intravenously, intramuscularly, or subcutaneously. For parenteral administration, these liposomes are best used in the form of a sterile aqueous solution which may contain other solutes, for example, sufficient salts, glucose or dextrose to make the solution isotonic.

For the oral mode of administration, the liposomes of the present invention can be used in the form of tablets, capsules, lozenges, troches, powders, syrups, elixirs, aqueous solutions and suspension,. and the like. In the case of tablets, carriers which can be used include lactose, sodium citrate, and salts of phosphoric acid. Various disintegrants such as starch, and lubricating agents may be used. For oral administration in capsule form, useful diluents are lactose and high molecular weight polyethylene glycols. When aqueous suspensions are required for oral use, certain sweetening and/or flavoring agents can be added.

Bioactive agents for use in the present invention may include but are not limited to those listed hereinabove, and include their pharmaceutically acceptable salts. Determination of compatibilities of the above listed agents with and the amounts to be utilized in compositions of the present invention are within the ordinary skill in the formulation art. The stability and applicability of individual pharmaceutical agents are well within the ordinary skill of practitioner in this art. It will be appreciated that the actual preferred amounts of bioactive agent utilized in a specific case may vary according to the severity of a pharmacological or disease condition and the expected pharmacokinetics of bioactive agent in the individual patient. Dosages for a given host can be determined using conventional considerations, e.g., by customary comparison of the differential activities of the subject bioactive agent by means of an appropriate, conventional pharmacological protocol.

IF liposomes can be remote loaded, for example, to incorporate bioactive agents. If desired, IF liposomes can be dehydrated using, for example, the procedures of Janoff et al. U.S. Patent No. 4,880,635 or Schneider et al. U.S. Patent No. 4,229,360.

The following examples are provided to illustrate the present invention and should not be construed to limit the scope of the invention of the present application in any way.

### EXAMPLE 1

Liposomes comprising dipalmitoylphosphatidylcholine (DPPC, obtained from Avanti Polar Lipids, Birmingham, Alabama, USA) were formed in 1 ml of an aqueous buffer solution to a concentration of 20 mM DPPC and additionally containing 0.04 mM diphenylhexatriene (DPH, purchased from Molecular Probes, Eugene, Oregon, USA). After formation of the liposomes, ethanol was added to a final concentration of 0.3 M to 2.5 M of the aqueous solution.

DPH fluoresces maximally when incorporated into the liposome bilayer. Interdigitation results in the reorientation of DPH from the bilayer membrane with a concomitant decrease of fluorescence. As shown in Figure 2, interdigitation is greater where higher concentrations of ethanol are present, for all liposomes. The effect of interdigitation by the same amount of ethanol is greater in those liposomes having a larger diameter. In Figure 2, Fo = DPH fluorescence in the absence of ethanol; F = DPH fluorescence in the presence of ethanol. Excitation = 351 nm. Emission was detected between 380 and 580 nm and quantitated by weighing.

### EXAMPLE 2

### Lipid Mixing of Liposomes

Lipid mixing of sized DPPC Liposomes was determined as a function of the size of the Liposomes and concentration of inducer. Liposomes comprising DPPC were formed in an aqueous buffer solution containing 20 mM DPPC. A marker population of liposomes containing 99% by weight DPPC, 0.35% by weight N-benzyldiphosphatidylethanolamine (MBD-PE) and 0.65% by weight rhodamine-phosphatidylethanolamine were formed in 1 ml. of an aqueous buffer solution. These probes form a donor-acceptor pair. The NBD moiety is excited at 465 nm and via resonance energy transfer (RET) becomes quenched by the rhodamine acceptor which itself becomes excited in a distance dependent phenomenon. These liposomes were mixed with blank liposomes at a 1:10 ratio. Emission spectra were recorded between 480 and 680 nm. Lipid mixing in DPPC liposomes of varying size as a function of ethanol concentration can be determined by the loss of RET from the NBD moiety to the Rhodamine moiety. A standard curve was generated by preparing liposomes of 0.35 mole percent NBD-PE and 0.65 mole percent Rhodamine-PE with sequentially decreasing these mole percents to 0.035 and 0.065 respectively. A direct comparison from the 1/10 mixing experiments with this standard curve indicates the degree of lipid mixing, an indication of membrane fusion.

### EXAMPLE 3

### Comparison of Trapped Solute in Various Vesicle Types

A number of liposomal formulations were prepared. The amount of trapped aqueous phase was determined and compared for each "type" of liposome prepared. The results appear in table 1, below.

For the preparation of IF liposomes, MLVs were prepared as described below to a final concentration of 20 µmoles DPPC per ml of aqueous buffer. The MLVs were then sonicated in a bath type sonicator at 50°C until translucent (SUVs). After the SUVs cooled to room temperature, ethanol was added to a final concentration of 2.0 M in the final aqueous suspension. For examples A1 and A2 (see table 1, below), ethanol was removed by dilution followed by washing. For B1 and B2, ethanol was removed via positive pressure displacement using N₂, after which the samples were diluted and washed. Sample C had one half the initial lipid concentration of A and B and ethanol removal was achieved as for sample A.

For the preparation of MLVs, 100 mg DPPC in 5 ml chloroform was rotary evaporated to a thin dry film in a round bottom flask to which a 1 ml aqueous buffer solution containing 0.04 mM diphenylhexatriene was added. Thereafter, the lipid mixture was vigorously vortexed until all lipid was removed from the wall.

FATMLVs were formed by subjecting unwashed MLVs as described above to 5 freeze and thaw cycles as described by Bally et al., U.S. Patent No. 4,975,282, issued December 4, 1990.

SPLVs were prepared by forming the thin dry film of DPPC as described above for MLVs and then dissolving the lipid film in 5 ml ethyl ether to which 0.5 ml of aqueous buffer was also added. This mixture was then emulsified in a bath type sonicator, a stream of N₂ was used to stir the emulsion while removing the ether as described by Lenk, et al., U.S. Patent No. 4,522,803. Ether removal was continued until no residual odor was detected (approximately five minutes). The resulting lipid mixture was resuspended in 1 ml aqueous buffer.

MPVs were formed as described in U.S. Patent No. 4,588,578, by preparing a monophase of 100 mg DPPC and 5 ml chloroform, 5 ml ethanol and 0.5 ml aqueous buffer, rotary evaporating to dryness, and resuspending the suspended film in 1 ml aqueous buffer by vigorous vortexing.

To determine the captured aqueous volume, 20 µl of a 10 mM 4-trimethylammonium TEMPO (4-TMAT) solution was added to 0.98 ml of the liposomal suspensions. The samples were then vortexed and the outer aqueous phase was separated from the liposomes by centrifugation. Because 4-TMAT neither binds to nor permeates the liposomes used in this study, it is concentrated in the outer aqueous phase. Measurement of 4-TMAT's concentration allows for calculation of the internal aqueous phase or captured volume as detailed in Perkins, et al., BBA, 943, 103 (1988). The results of this analysis appear in Table 1, below. As seen, the IF liposomes sequester significantly greater volumes, in some cases as much as 10 times that attained with the other liposome types.

**Table 1**

| Comparison of Trapped Solute | | |
|---|---|---|
| Liposome Type | | *Captured Volume (µl/µmole) |
| IF | A1 | 6.7 |
| | A2 | 7.8 |
| | B1 | 8.3 |
| | B2 | 7.2 |
| | C | 8.9 |
| MLV | 1 | 0.56 |
| | 2 | 0.78 |
| MPV | 1 | 0.71 |
| | 2 | 1.9 |
| SPLV | 1 | 2.0 |
| | 2 | 2.8 |
| FATMLV | 1 | 2.7 |
| | 2 | 2.6 |

| | | |
|---|---|---|
| *Captured volumes were measured using the EPR technique (Perkins, et al. (1988) Biochim. Biophys. Acta 943, 103) where the samples are examined after formation. | | |

### EXAMPLE 4

### Procedure for Formation of IF Liposomes

Liposomes (LUVs, MLVs, SPLVs, etc. as prepared above) comprising a saturated lipid such as dimyristoylphosphatidylcholine, dipalmitoylphosphatidylcholine or distearoylphosphatidylcholine are prepared and sized to 0.4 µm (microns) or less (preferably, 0.025 µm (microns)) by extrusion, sonication or homogenization. A bioactive agent which does not interact with the lipid is then mixed in with the aqueous solvent used to form the Liposomes (final lipid concentration should be about 5 to 100 mM, preferably about 10 to 35 mM). The temperature of the liposomes are below the main phase transition temperature of the lipid. Thereafter, ethanol is added to a final concentration of about 1.75 to about 2.5 M in the aqueous solvent. In the case of bioactive agents which interact with the lipid, the agents are generally added to the solvent after the addition of ethanol and formation of the IF gel. At this stage, the procedure can be stopped and the resulting gel used in topical and oral formulations. Alternatively, the gel may be used to form IF liposomes of the present invention.

To form IF liposomes, the gel is incubated at a temperature below the Tm of the lipid for a period ranging from about 1 minute to about 1 hour followed by an incubation period of about 1 minute to about 1.0 hour at a temperature above the Tm of the lipid. The inducer is then removed by evaporation, positive nitrogen pressure or dilution. In the case of ethanol, the ethanol may be diluted to a concentration below about 0.2 M. As the inducer is removed, liposomes form, generally varying in size from about 0.25 µm (microns) to about 20 µm (microns).

### EXAMPLE 5

### Scale-up of Diatrizoate-DPPC IF Liposomes

600 mg of DPPC were dried to a film from chloroform by rotary evaporation, then dried further under vacuum for 16 hours. The lipid was resuspended in 6 ml of 0.9% saline by incubation in a 51°C bath for approximately 15 minutes. The resulting multilamellar liposomes (MLVs) were transferred to a 30 ml Corex tube and bath sonicated for two hours at 51°C until the solution was translucent. At this point only 4.1 ml of liposome suspension could be retrieved. 12 ml of diatrizoate (Renografin-76™, available from Bristol-Myers Squibb), 12 ml of deuterated water ("dH₂O"), and 2 ml of ethanol were mixed in a 50-ml centrifuge bottle to which the 4.1 ml lipid (still at 51°C) was added and then briefly vortexed. Within 10 minutes the mixture became a loose, pourable gel which was allowed to set at room temperature for 2 hours. Next, the suspension was incubated in a 51°C bath for 1 hour. At this point the sample was split in half to facilitate ethanol evaporation. While still immersed in the bath, N₂ was bubbled through each aliquot for a period 12 minutes. The samples were allowed to cool to room temperature followed by dilution with 20 ml of 0.9% saline per aliquot. The preparation was washed by repetitive centrifugation at 10,000 x g at 20 C for 10 minutes for 3 cycles. The sample was assayed as described for Example 6 below. The results appear in Table 2, below.

**Table 2**

| Aliquot Weight (mg) | I:L | % Entrapped | mg/ml Iodine | Pellet |
|---|---|---|---|---|
| A | 2.4 | 16%¹ | 48.8 | 7.42 |
| B | 3.8 | 25% | 67.6 | 8.07 |

| | | | | |
|---|---|---|---|---|
| 1- Based on 12 ml. of diatrizoate initially added. | | | | |

### EXAMPLE 6

### Diatrizoate-HSPC IF Liposomes

400 mg HSPC (Natterman Phospholipids) was hydrated at 70 C in 10 ml dH₂O for about 1 hour and probe sonicated until translucent (30 minutes). 14 ml diatrizoate (Squibb) containing approximately 1 µCi/ml 125I-diatrizoate was mixed with 2.1 ml dH₂O and 4.9 ml ethanol in a 50 ml screw-top Corex™ tube. 7 ml of the HSPC small unilamellar liposomes (SUV) was added while mixing; this was done while the SUV were still above their Tm. A solid gel formed immediately and was allowed to set at room temperature, covered for approximately 1 hour. The preparation was then incubated for 2 hours in a 70°C water bath, uncovered after which N₂ was bubbled through the now liquid prep for 23 minutes. The N₂ flow rate was at 10 on the Manostat™ flowmeter. A 4 ml aliquot was dispensed into a 30 ml Corex™ tube and allowed to cool to room temperature. 10 ml of 900 mOsm meglumine buffer (prepared from meglumine, NaCl, citrate, EDTA) was added and the preparation was vortexed briefly. The unentrapped diatrizoate was removed by repetitive centrifugation at 10000 X g at 18°C for 15 minutes for 3 cycles. The final iodine concentration was determined by extrapolation from a UV spectrophotometric assay of diatrizoate (A256) to be 106.3 mg/ml. The final lipid concentration was 12.1 mg/ml as determined by the standard methodologies described by Chen, et al., Analytical Chem., 28, 11, 1756 (1956). The final I:L ratio was 8.8 (w/w). The resulting liposomal suspension was stored under ambient conditions.

### EXAMPLE 7

### Iotrolan-HSPC IF Liposomes

1 g HSPC (Natterman Phospholipids) was hydrated at 70°C in 25 ml dH₂O for about 1 hour and probe sonicated until translucent (about 30 minutes). The SUVs produced were transferred to a 50 ml screw-top Corex™ tube and spun at 5000 X g for about 5 minutes in order to pellet any titanium present. The SUVs were decanted from the titanium pellet and incubated in a 70°C water bath for about 5 minutes before being added to the following solution: 44 ml ¹²⁵I-labeled Iotrolan was mixed with 15.6 ml ethanol and 6.4 ml dH₂O. This solution was then divided into 4 X 16.5 ml aliquots in 50 ml screw-top Corex™ tubes. 5.5 ml SUVs were added to each tube while mixing resulting in the formation of loose gels. The gels sat covered for 1 hour at room temperature, then each was transferred to a 70°C water bath for 1 hour, uncovered, after which N₂ was bubbled through the liquid in each tube for 13 minutes at a flow rate of 40 on the gas flowmeter. Each tube was emptied into a 250 ml Erlenmeyer flask where it cooled to room temperature. About 150 ml sterile PBS (phosphate buffered saline without Ca and Mg, pH 7) was added while swirling the flask. The unentrapped Iotrolan was removed by repetitive centrifugation at 10000 X g for 10 minutes at 18°C for 3 cycles. The final iodine concentration was based on the initial specific activity of the Iotrolan solution at 300 mg/ml iodine and was found to be 152.7 mg/ml. The final phospholipid concentration was determined to be 32.7 mg/ml by the method of Chen, et al., Analytical Chem., 28, 11, 1756 (1956) and was corrected for the presence of phosphate in the buffer. These values resulted in a final I:L ratio of 4.7 (w/w). The resulting liposome suspension was stored under ambient conditions.

### Example 8

### Shelf Stability of Radiocontrast Agent IF Liposomes Stored Under Ambient Conditions

50 µl of either diatrizoate or iotrolan HSPC IF liposome preparations (radiolabeled) were diluted with 1 ml of their original suspension buffer and centrifuged at 16,000 X g in a microfuge at room temperature for 10 minutes. The supernatants were removed and both the pellets and the supernatants counted for 125I. After 62 days at 25°C, there was 4% of the radiolabel in the supernatant of the diatrizoate liposomes and after 54 days, 3% in the supernatant of the iotrolan liposomes. Based upon these results, the preparations exhibit a shelf life of at least about 1 year when stored under ambient conditions.

### EXAMPLE 9

### Effect of Initial Lipid Concentration on Entrapment of Diatrizoate in DPPC IF Liposomes

The results which appear in Table 3, below represent IF liposome preparations made as previously described In Example 5 with variation in the initial lipid concentration. Briefly, in 15 ml. Corex tubes, 1 ml diatrizoate was mixed with dH₂O and DPPC sized unilamellar Liposomes (SUVs) at 50 mg/ml to achieve the final volumes shown below. Before lipid addition, 87.5 µl ethanol per ml. (final volume 2M) was added to the diatrizoate solution. Preparations sat, capped, for 1 hour at room temperature followed by incubation in a 51°C bath, uncapped. After 1 hour N₂ was bubbled through each sample for 2 minutes. After cooling to room temperature, each sample was diluted with 10 ml of 0.9% saline solution and washed by repetitive centrifugation at 10,000g for 15 minutes at 20°C for 3 cycles. The samples were assayed as previously described (Example 3). The results appear in Table 3, below.

**Table 3**

| mg.DPPC/ Total ml | I:L | % Entrapped¹ | mg/ml pellet Iodine | Weight |
|---|---|---|---|---|
| 50 mg/ | 1.7 | 38 | 57.6 | 2.47 |
| 2 ml. | 1.8 | 35 | 58.9 | 2.18 |
| | | | | |
| 25 mg/ | 3.5 | 37 | 68.7 | 2.01 |
| 2 ml. | 3.1 | 28 | 62.1 | 1.66 |
| | | | | |
| 25 mg/ | 5.0 | 49 | 73.5 | 2.47 |
| 3 ml. | 5.6 | 56% | 80.3 | 2.56 |

| | | | | |
|---|---|---|---|---|
| 1- Based on 1 ml diatrizoate initially added. | | | | |

### Example 10

### Entrapment of Gentamicin Via IF Liposomes

A solution of Gentamicin sulfate (Sigma Chemicals, St. Louis, Missouri, USA) was prepared in 0.9% saline solution to a final concentration of 500 mg/ml. Separately, 100 mg of DPPC (Avanti Polar Lipids) was evaporated to dryness and hydrated in 2.5 ml saline to a final concentration of 40 mg/ml. The DPPC mixture was sonicated on a bath sonicator to clarity.

The following mixtures were then made. Note that the ethanolic inducer was added before the solutions containing gentamicin sulfate.
A - 0.5 ml. DPPC solution, plus ethanol ("EtOH") (to a final concentration of 2M) and 0.5 ml. gentamicin solution;
B - 0.25 ml. DPPC solution, plus 0.25 ml 0.9% NaCl saline solution, EtOH (to a final concentration of 2M) and 0.5 ml. of gentamicin solution;
C - 0.25 ml. DPPC solution, plus 0.75 ml saline, EtOH (to a final concentration of 2M) and 1.0 ml. of gentamicin solution;

Each of the above preparations were then assayed for gentamicin activity by the agar well diffusion bioassay to determine lipid:gentamicin concentration. In brief, the liposomes in each of the above preparations were then disrupted with 0.2% Triton-X 100 (Biorad Laboratories, Richmond CA) and assayed for gentamicin activity using an agar well diffusion bioassay with Bacillus subtilis (ATCC #6633) as the indicator organism. Lipid concentration was determined by standard methods described by Ames, et al., Journal of Biological Chem. 235, 236, 769 (1960).

The results of the bioassay determinations appear in Table 4, below. The results indicate that very low lipid/gentamicin weight ratios may be obtained.

**Table 4**

| Entrapment of Gentamicin in IF Liposomes | | | |
|---|---|---|---|
| Sample | Gentamicin¹mg/ml | Lipid mg/ml | Lipid/Gentamicin Weight Ratio |
| A1 | 6.18 | 19 | 3.07 |
| A2 | 3.13 | 12.5 | 4.0 |
| B1 | 2.16 | 13.2 | 6.11 |
| B2 | 3.15 | 11.0 | 3.49 |
| C1 | 4.25 | 8.6 | 2.02 |
| C2 | 2.46 | 6.05 | 2.46 |

| | | | |
|---|---|---|---|
| 1- Corrected for Potency (616 µg/mg). | | | |

### Example 11

### Scale up of DSPC - Iotrolan IF Liposomes

Eight (8) grams of DSPC were mixed in 200 ml of Water for Injection ('WFI") at 70°C for 30 minutes. The resulting suspension was passed through a Microfluidizer™ homogenizer 25 times at a pressure of 758.34 x 10⁵ Pa (11 000 psi) thereby forming SUVs. The resulcing SUVs were filtered through a 0.22 µm pore size Millipore™ tortuous path polymeric filter.

Iotrolan (92 ml, at 300 mg/ml). 13.4 ml WFI, and 32.6 ml ethanol were admixed in a 2,000 ml capacity round bottom flask. The SUVs (44 ml) were admixed in the flask at room temperature (about 25°C) and mixed using a banana paddle mixer for 10 seconds. The gel which formed thereafter mixing was allowed to sit undisturbed for 1.25 hours.

The round bottom flask was placed in a 70°C water bath and mixed using the banana paddle at 66 rpm for one (1) hour. Ethanol was then removed by nitrogen sparge over the aqueous surface at a rate of 4.7 L N₂/minute for one (1) hour, collecting the ethanol in a trap, with the mixing increased to about 135 rpm. The final volume was adjusted to about 400 ml with carbonate buffer (0.4 mg/ml NaHCO₃, 0.1 mg/ml disodium EDTA, in 0.9% saline). The resulting IF liposomes were washed, thereby separating liposomes from unentrapped Iotrolan by diafiltration through a 0.2 µm Microgon filtration device. Seven washes of 100 ml were employed with removal of 300 ml last, as a concentrating step. This washing step proceeded for 25 minutes.

Analysis of the resulting Iotrolan/DSPC liposomes yielded the following results shown in Table 5, below:

**Table 5**

| | |
|---|---|
| Lipid concentration | 23.0 mM, 18.2 mg/ml |
| Lyso PC content | 0.9% |
| Iotrolan entrapped | 264.8 mg/ml |
| Free Iotrolan | 1.4% |
| Iotrolan/DSPC | 14.5 |
| Captured Volume | 13.7 µl/µmole |
| Size distribution | 90% less than 3.6 µm |
| | 50% less than 2.8 µm |
| | 10% less than 1.2 µm |

### EXAMPLE 12

### Encapsulation Efficiency of DPPC- IF Vesicles as a Function of Ethanol Concentration

DPPC (powder) was mixed with 10 mM Tris HCl, 150 mM NaCl also containing a trace amount of ¹⁴C sucrose, at pH 7.4 to a concentration of 20 mg/ml DPPC, for a total of 2.0 ml. The mixing was done at a temperature above the phase transition temperature of DPPC, at 50°-53°C, and resulted in MLVs. The MLVs were sonicated for one hour at 50-53°C and cooled to room temperature, resulting in SUVs of about 30-50 nm in diameter. To the 2.0 ml of SUVs was added enough ethanol (100%) to result in a 3.0 M ethanol concentration (0.43 ml ethanol; 20% ethanol by weight total); the mixture was vortexed to homogeneity. The suspension was allowed to sit capped and undisturbed for one hour at room temperature, then was incubated one hour at a temperature above the transition temperature of DPPC i.e., at 50°-55°C, with the cap loosened. While still incubating above the lipid Tₘ, a gentle stream of N₂ was bubbled through the mixture for 3 minutes. A sample (100 µL) was removed for a ¹⁴C sucrose encapsulation study, and a 4 µL and 8 µl aliquot also removed for determination of Pi according to the Bartlett phosphorous assay of Chen et al. Ten ml of Tris/NaCl buffer was added to the resulting IF liposomes, and the mixture was centrifuged at 9,000 x g for 15 minutes, the supernatant decanted and the pellet resuspended in Tris/NaCl buffer and centrifuged two additional times for a total of 3 washes. The pellet was finally resuspended in 2.0 ml buffer. Another 200 µL sample was removed for the encapsulation study, as well as 4.0 µL and 8.0 µL aliquots for the Pi assay.

The above method was repeated using a total ethanol concentration of 1.0, 2.0, 2.5, 3.5, and 4.0 M in solution.

The internal volume of the IF liposomes is expressed as µL per µM of phosphorous Pi, and was measured by ¹⁴C sucrose encapsulation and CAT 1 EPR methods.

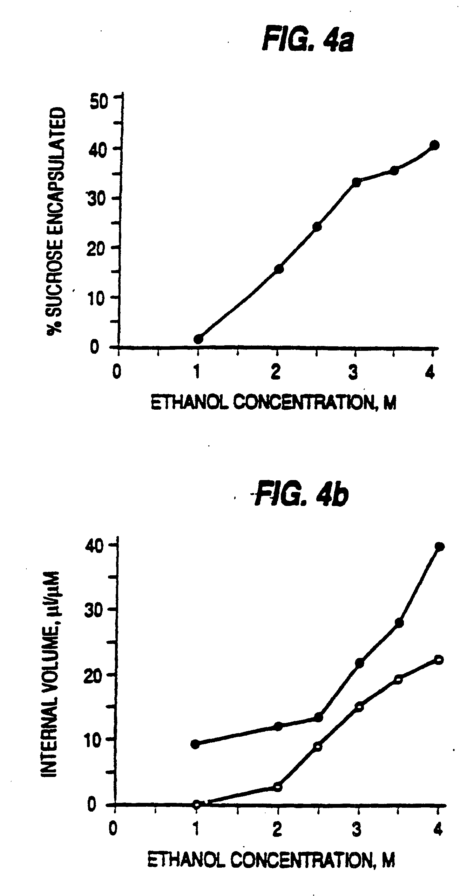

The ¹⁴C sucrose encapsulation was performed as follows: Following the N₂ bubbling step, the 100 µL sample removed for the ¹⁴C sucrose encapsulation study was counted in a Beckman model Ls 6800 scintillation counter. Similarly, following the centrifugation step, the 200 µL sample removed was centrifuged at 3,000 x g using a table top centrifuge, and both the pellet and the supernatant was counted in the scintillation counter. In addition, the Pi was determined as before. The ¹⁴C sucrose encapsulation was thereby determined and the results are represented graphically in Figure 4a.

The CAT 1 EPR study was performed as in Example 13 hereinbelow.

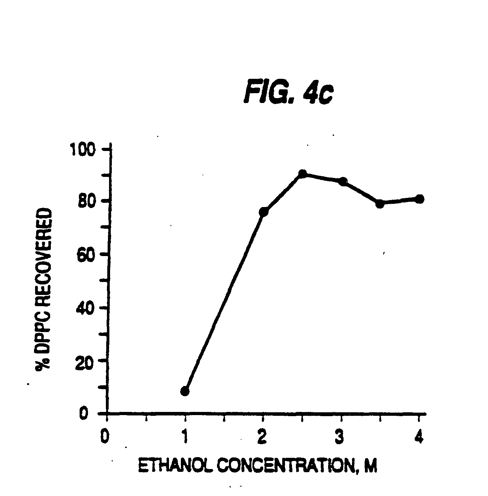

As shown in Figure 4c, the internal volume of the DPPC IF liposomes increased as a function of increased ethanol concentration, consistent with higher encapsulation efficiency of liposomes at higher ethanol concentrations. The percentage of DPPC recovered following the three centrifugation washes is shown in Figure 4c.

### EXAMPLE 13

### Encapsulation Efficiency of DSPC, DHPC, DOPC and EPC IF Vesicles as measured by sucrose and EPR Methods

The methods of Example 12 were repeated using the lipids DSPC, DHPC, DOPC and EPC, using a final concentration of 3.0 M ethanol. The low temperature incubations following ethanol addition were done at 50°C for DOPC and EPC. The high temperature incubation was done at 70°C for all samples.

Incubation of DSPC took place at 70°C, DHPC at 50°C, and DOPC and EPC at 5°C.

The DOPC and EPC samples were not washed by centrifugation but filtered through an Amicon™ 30K microconcentration device (Grace Co.) filters. 100 µL aliquots were removed from the filtrate and from the sample before and after the filtration for the entrapment efficiency analysis.

Entrapment efficiency was calculated by the sucrose encapsulation, CAT 1 EPR and TEMPONE EPR methods, methods for performing all of which are recited hereinbelow.

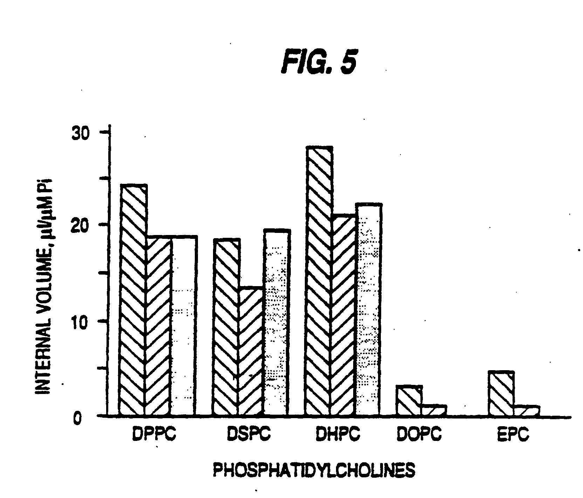

As shown in Figure 5, the three phosphatidylcholines which are known to interdigitate (DPPC, DSPC and DHPC) all produced IF liposomes with high internal volumes. EPC and DOPC had relatively low internal volumes, were relatively small and could not be pelleted using a table top centrifuge (9,000 x g). Therefore it was not possible to measure internal volumes of these liposomes using the CAT 1 EPR method.

### CAT 1 EPR METHOD FOR DETERMINING ENTRAPMENT

This method is alternatively known as the external solvent volume method, described in Perkins et al., 1988, Biochim. Biophys. Acta, 943:103-107. The internal volume of liposomes was determined by subtracting the external solvent volume, calculated by measuring the concentration of a membrane impermeant spin probe from the total volume of the liposome suspension. The external solvent volume was calculated by adding a known amount of a spin probe 4-trimethylammonium-2,2,6,6-tetramethylpiperidine-1-oxyl iodide ("CAT 1") to a liposome suspension. The liposome suspension was centrifuged to pellet the liposomes. The concentration of CAT 1 in the solvent was determined by comparison of the magnitude of the CAT 1 EPR signal from the supernatant to an EPR signal versus CAT 1 concentration calibration curve. The CAT 1 concentration in the supernatant was higher than what would be expected based on the amount of CAT 1 added because the volume available to the spin probe was reduced as the probe was excluded from the inside volume of the liposomes. Correction was also made for the sample volume due to the lipids themselves.
Procedure: A stock solution containing 10 mM CAT 1, 10 mM Tris HCl (pH 7.4), 150 mM NaCl was used for the calibration buffer solutions containing 100, 200, 300 and 400 µM CAT 1. The EPR spectrum of each stock solution was recorded with a Brucker ER 100D spectrometer. The peak to peak height of the M_{I}=+1 resonance line of each spectrum measured for each concentration and a peak height versus CAT 1 concentration curve was constructed.
CAT 1 stock (0.20 µM) was added to 1.00 ml of the liposome suspension (Vₜ) and vortically mixed. The liposomes were pelleted by centrifugation on a table top centrifuge at 9,000 x g and a small portion of the supernatant was drawn into an EPR capillary tube and sealed. The peak to peak height of the M_{I}=1 resonance line was measured. The solvent concentration of CAT 1 was determined from the magnitude of the sample EPR signal and the calibration curve.
The external solvent volume Vo was obtained by the calibration curve. The external solvent volume Vo is equal to M/C where M is the moles of CAT 1 added and C is the CAT 1 concentration in the supernatant. V1 is the volume occupied by the lipid, is expressed as Vi = 1.00-Vt-V1, where V1 is the volume of the lipid. The internal volume, Vi, divided by the phosphate content of the sample gives the internal volume per µM Pi which is the standard way of expressing the internal volume of liposomes.

### TEMPONE EPR METHOD FOR DETERMINING ENTRAPMENT

This method is alternatively known as the broadening agent method, described in Anzai et al., Biochim. Biophys. Acta 1988, 937:73-80. The internal volume of liposomes is determined by measuring the amount of membrane permeant EPR spin probe broadening agent. Normally rapidly tumbling aqueous EPR spin probes have relatively narrow spectral line shapes, however, addition of paramagnetic ions decreases the spin-spin relaxation time (T₂). If the broadening agent is at high enough concentration it can drastically broaden the spectral line shape and dramatically decrease the peak to peak height of EPR signals. In effect, if the EPR broadening agent has access to the spin probe, the probe signal is eliminated.

The measurements are done by adding the membrane permeant EPR spin probe 4-oxo-2,2,6,6-tetramethylpiperidine-1-oxyl ("TEMPONE") to liposomes suspensions. The two 200 µl aliquots were removed from each liposome suspension. One of the aliquots was diluted with 200 µl buffer while the other was diluted with 200 µl of buffer plus the membrane impermeant broadening agent potassium tris(oxalato)chromate (III). The EPR signal from the aliquot diluted with buffer is proportional to the total sample volume, while the EPR signal from the aliquot diluted with the potassium tris(oxalato)chromate (III) is proportional to the sample volume inside the liposomes.
Procedure: A stock solution of 50 mM TEMPONE, 10 mM Tris-HCl (pH 7.4), 150 mM NaCl stock solution was prepared. TEMPONE stock (10 µl) was added to 0.5 ml suspension of liposomes and vortically mixed. A 200 µl aliquot was taken from each sample and diluted with 200 µl of buffer. Another 200 µl aliquot was taken and diluted with a 100 mM potassium tris(oxalato)chromate (III), 50 mM NaCl solution. The osmotic strength of the buffer and the chromate solutions were previously checked with a vapor pressure osmometer to insure that they were equal to within 1-2%. The samples were vortically mixed.
The EPR spectra were recorded with a Brucker ER 100D spectrometer. Samples were drawn into EPR capillary tubes and sealed. The EPR spectrum of the buffer sample was recorded first, the chromate solution sample was then prepared, and the EPR spectrum of this sample was then immediately recorded. The peak to peak height of the M_{I}=-1 resonance line was used to measure the relative concentrations of the unaffected spin probe in both samples. The total sample volume was proportional to the EPR signal size of buffer diluted aliquot divided by the spectrometer gain setting (ST), while the internal volume of the liposomes was proportional to the EPR signal of the aliquot diluted with chromate solution divided by the gain setting (SI). The internal sample volume (Vi) was given by the SI/ST times the sample volume V. The internal volume, Vi, of the sample was divided by the phosphate content of the sample to give the internal volume per µM Pi which is the standard way of expressing the internal volume of liposomes.

### EXAMPLE 14

### Encapsulation Efficiency of DPPC LUVET - IF Vesicles as a Function of Initial Size of Liposomes

DPPC (422 mg powder) was hydrated with 21 ml of Tris/NaCl with a trace amount of ¹⁴C sucrose according to the methods of Example 12, for a total concentration of DPPC of 20 mg/ml. Vortical mixing of the sample at 50-55°C resulted in DPPC MLVs. FAT MLVs were prepared according to the methods of Cullis et al., U.S. Patent No. 4,975,282, issued December 4, 1990, for a total of 10 freeze and thaw cycles. The resulting DPPC FATMLVs were extruded ten times using the LUVET apparatus at 60°-65°C according to the methods of Cullis et al. PCT Application PCT/US85/01161, Publication Number WO 86/00238 entitled "Extrusion Techniques for Producing Liposomes", published January 16, 1986, and employing a single 1.0 µm Nuclepore polycarbonate filter. Two ml of the LUVET processed liposomes were set aside.

The above method was repeated employing 0.4, 0.2, and 0.1 µm polycarbonate filters, until 2.0 ml LUVET samples of the following diameters were produced: 0.1, 0.2, 0.4, and 1.0 µm.

When FAT MLVs were employed for the purposes of this Example, they were used as is, without extrusion. SUVs were prepared according to the methods of Example 12 by sonication of the remaining 0.1 µm filtered LUVETs.

Internal volumes of these liposomes were calculated by ¹⁴C sucrose encapsulation as well as CAT 1 EPR and TEMPONE EPR methods as described hereinabove.

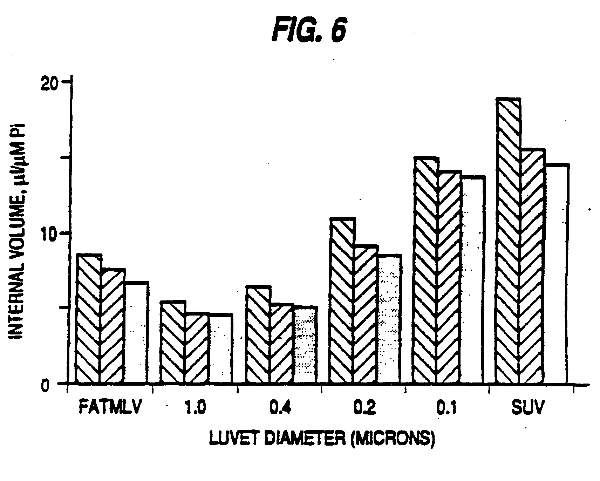

As shown in Figure 6, except for FATMLVs, the internal volume of the IF liposomes increased with decreased size of "starting" liposomes e.g., liposomes prior to the addition of ethanol; these results indicating that the diameter of the starting liposome was an important parameter in determining the final volume of the IF liposome.

### EXAMPLE 15

### Formation of Interdigitation-Fusion Vesicles

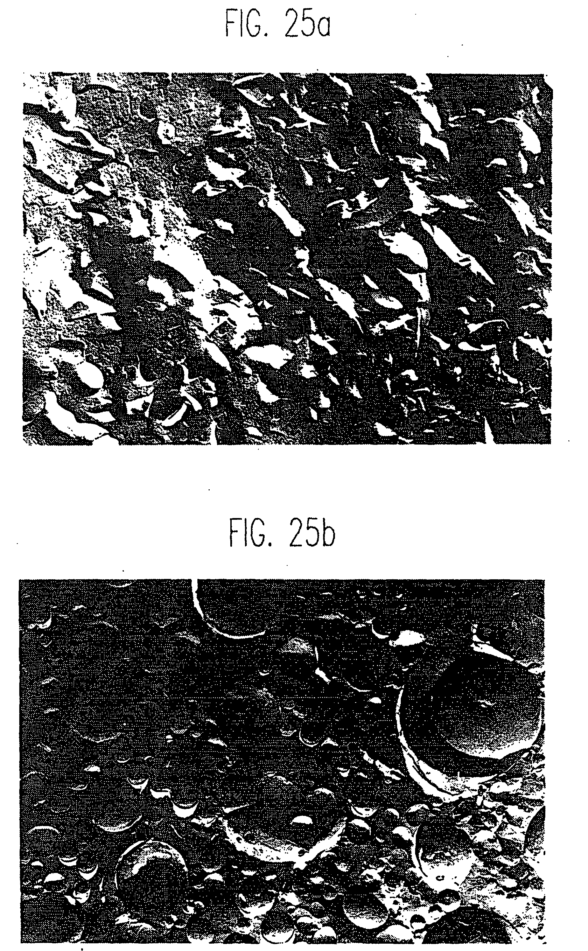

Interdigitation-fusion vesicles (IFVs) can be formed from phospholipids or combinations of phospholipids which undergo interdigitation, such as DPPC or DSPC, e.g., by inducing interdigitation-fusion of small unilamellar vesicles (SUVs) comprising such phospholipids. The coalescence of DPPC SUVs into interdigitated sheets, and the transformation of these sheets into IFVs, is shown in Figures 24 and 25 by optical phase contrast microscopy and freeze-fracture electron microscopy, respectively. Optical phase contrast microscopy was carried out using a Leitz Laborlux D micrscope in combination with a Wild MPS45 exposure meter. Kodak GC 135-24 color print film was used to record the images. The samples were observed at room temperature. Freeze-fracture electron microscopy was carried out by sandwiching a 0.1 to 0.3 microliter aliquot of sample between a pair of Balzer copper support plates (Nashua, NH) and then rapidly plunging the sample from room temperature into liquid propane. Samples were fractured and replicated on a Balzers BAF 400 freeze-fracture unit at minus 115 degrees Celsius with a vacuum of 4 x 10⁻⁷ mbar. Replicas were floated off in 3N nitric acid and washed in a series of Clorox solutions. The replicas were viewed and photographed in a Philips 300 electron microscope at magnifications of between 6,000X and 27,000X.

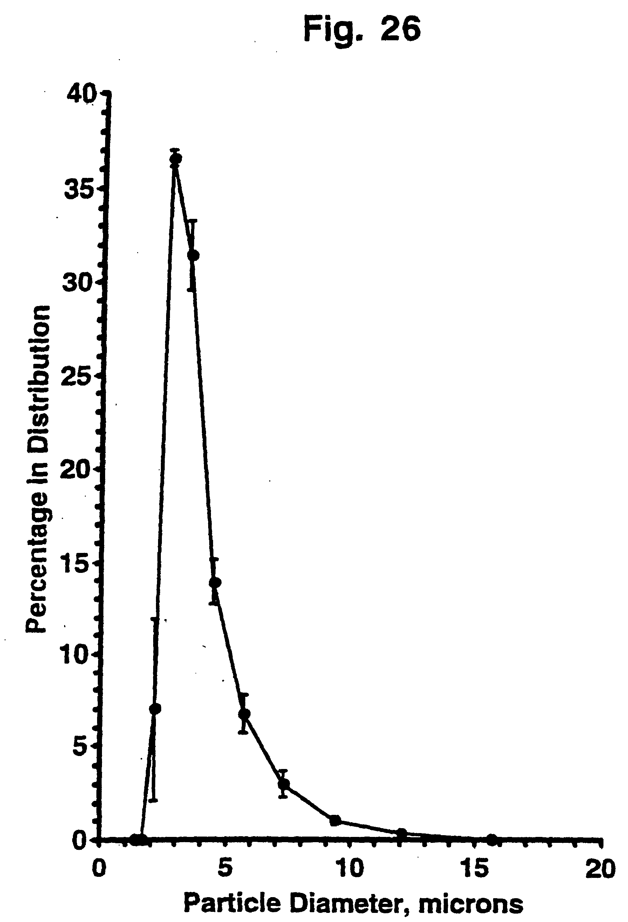

Formation of interdigitated sheets from the DPPC SUVs occurred immediately upon the addition of ethanol. The optically clear SUV suspension was quickly transformed into an opaque, highly viscous fluid. The interdigitated sheets that comprised this opaque suspension had dimensions on the order of hundreds of microns as indicated in Figure 24a. Raising the temperature above the Tm of DPPC quickly transformed the interdigitated sheets into relatively large unilamelar vesicles. The number averaged size distribution of DPPC IFVs formed at 20 mg/ml with 4.0M ethanol is shown in Figure 26. The distribution was relatively homogeneous, with most of the liposomes being in the 2 to 6 micron range. The internal volume of the DPPC IFV preparations, averaged to obtain the data shown in Figure 26, was 20.2 +/- 0.2 µl/µ M, while the NMR lamellarity measurements indicated 44.3+/-12.5 of the DPPC was in the outer monolayer of the liposomes. This corresponds to a statistical lamellarity of 1.13+/-0.32. Thus, the DPPC IFVs were predominantly unilamellar.

The effects of varying ethanol concentration, precursor vesicle diameter, and lipid concentration upon the internal volume of DPPC IFVs are shown in Figure 27. Figure 27a shows the effect of varying the concentration of ethanol used to produce the interdigitated sheets from the DPPC SUVs. The internal volume was measured using the ESR spin probe CAT-1 as previously described. For ethanol concentrations of 3.0M and above, the DPPC IFVs were separated from the solvent by centrifugation. At ethanol concentrations of 2.0M and below, the liposomes were separated from the solvent by filtration through a Whatman 0.02 micron Anotop 25 filter (Whatman, Inc., Clifton, NJ). This filter was chosen because the cationic spin probe CAT-1 does not bind to it.

The internal volume of DPPC IFVs increased linearly from ethanol concentrations of 2.0M to 4.0M. There was no significant difference in either average particle diameter or the lamellarity of DPPC IFVs formed at 2.0M ethanol compared with those formed at 4.0M. This suggests that the observed internal volume increase was due to differences in the shapes of the liposomes. IFVs were not formed efficiently at ethanol concentrations greater than 5.0M.

Figure 27b shows how the interdigitation-fusion process depends upon the diameters of the precursor DPPC liposomes. The ethanol concentrations in the experiments whose results are summarized in the Figure was held at 4.0M, which appeared to be near the optimum concentration for obtaining high internal volumes with DPPC SUVs. The diameter of precursor DPPC LUVETs was determined by quasi-electric light scattering using a Malvern 3600 E laser diffraction particle sizer (Malvern Instruments, Malvern, England). The measurements were made at room temperature with highly diluted samples. The number weighted particle diameter distribution was calculated by the instrument rom the laser diffraction pattern. Precursor vesicles below 100 nm yielded DPPC IFVs of having the largest internal volumes. Large IFVs were not observed unless the dimater of the precursor vesicles was 150 nm or less. Vesicles 200 nm or more in size were relatively ineffective as precursor liposomes.

The effect of initial DPPC SUV concentrations on the internal volumes of the IFVs formed at 4.0M ethanol is shown in Figure 27c. As indicated, the internal volume decreased as the lipid concentration in the interdigitated sheet suspension was raised above 10 mg/ml. At higher lipid concentrations, the vesicles can be expected to be tightly packed as the ratio between internal and external aqueous volumes becomes limiting. The packing problem resulted in a significant lamellarity increase for the DPPC IFVs formed at high lipid concentrations. The statistical lamellarity for DPPC IFVs formed at 100 mg/ml was 2.9, in comparison to a value of 1.1 for DPPC IFVs formed at 20 mg/ml.

Using paramters optimized for DPPC, IFVs were produced using a variety of saturated phospholipids (DMPC, DHPC, DSPC and DAPC (Avanti Polar Lipids, Alabaster, AL)). IFVs were typically prepared in 4 ml batches using a lipid concentration of 20 mg/ml. Samples containing two lipids were prepared at 30 mM total lipid. The vesicles were prepared in containers such as scintillation vials or tubes with caps. An SUV suspension of the desired phospholipid was transferred into the container. DPPC, DSPC, DHPC and DAPC samples were equilibrated at room temperature, while DMPC, DOPC, and EPC samples were cooled to between 4 and 6 degrees Celsius. An isothermal volume of absolute ethanol was added to bring the final ethanol concentration in the sample up to 4.0M. The samples were then immediately vortexed. This procedure quickly transformed the transparent SUV suspension into an extremely viscous opaque white suspension of phospholipid sheets. The ethanol addition step was modified when the final ethanol concentration used to induce intedigitation was 1.5M or less. For such samples, equal volumes of the SUV samples at 40 mg/ml and a buffer/ethanol solution at twice the desired final ethanol concentration were mixed. This procedure was required to avoid having locally high ethanol concentrations in the SUV sample before it could be completely mixed. This effect produced a high internal volume mixing artifact at the low ethanol concentrations. No difference in the internal volume of the DPPC IFVs formed at the 2.0M ethanol concentrations was observed between the direct addition of the ethanol versus the addition of prediluted ethaol.

After ethanol addition, the samples were sealed and incubated at room temperature for 15 minutes, except for the DMPC, DOPC and EPC samples, which were incubated at 4-6 deg. C. The caps on the samples were loosened and the samples were then incubated for another 30 min. at the same temperature above the Tm. DMPC, DOPC and EPC samples were incubated at 40-45 degrees C. DPPC and DHPC samples were incubated at 50-55 deg. C, while DSPC and DAPC samples were incubated at 70-75 deg. C. The ethanol was typically removed by a two-step procedure involving first sparging the samples by bubbling a gentle nitrogen stream through the sample above the lipid's Tm. The samples were then washed three times at room temperature by 15 min. centrifugation at 12,000 g using a Sorvall RE5B centrifuge (DuPont Instruments, Wilmington, DE) using a Sorvall SA-600 rotor. This was sufficient to rapidly pellet the IFVs. Typically, 90-100% of the initial phospholipid was recovererd in the IFV pellet when 3.0-4.0M ethanol was used to prepare the interdigitation-fusion sheets. The IFV pellet was subsequently resuspended in NaCl/Tris buffer at a concentration near 20 mg/ml and stored at room temperature. IFV phospholipid concentraions were determined by a modified Bartlett assay ((Bartlett, J. Biol. Chem. 234:466 (1959)).

The same general procedures were used to prepare IFVs from two phospholipids. Ethanol was added below the Tm of the mixture; the high temperature incubation was also performed above the Tm of the mixture.

Incorporation of cholesterol into DPPC IFVs was done by adding 1:1 molar ratio DPPC/Chol SUVs in 4.0M ethanol to DPPC interdigitated sheets also at 4.0M ethanol. The interdigitated DPPC sheets were prepared from DPPC SUVs at a concentration of 30 mM using 4.0M ethanol, according to previously described procedures. DPPC/Chol SUVs in 4.0M ethanol were mixed into the interdigitated sheets at room temperature in such a manner as to give the desired Chol:DPPC mole ratio with a final lipid concentration of 30 mM. The mixtures were then incubated for 45 min. at 50-55 deg. C and sparged with nitrogen above the lipid Tm for 5 minutes to remove the ethanol. Since the size of the product DPPC/Chol IFVs decreased with increasing cholesterol concentration, the centrifugation/wash step was omitted. Control experiments showed that omitting this step did not affect the final internal vesicle volume.

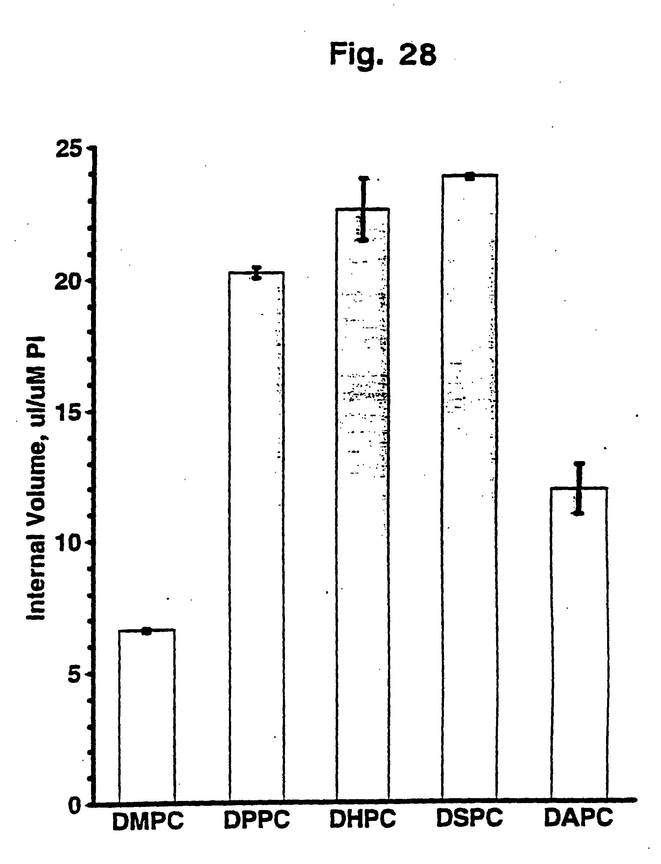

Figure 28 shows the internal volumes of IFVs produced from DMPC, DPPC, DHPC, DSPC and DAPC SUVs. The internal volumes of vesicles formed from phospholipids which do not undergo ethanol-induced interdigitation-fusion , e.g., EPC and DOPC, were less than 1 microliter/µM Pi.

The interdigitation-fusion technique was very effective as applied to SUVs comprising two or more phospholipids which can undergo ethanol-induced interdigitation-fusion. For example, the internal volume of IFVs formed from DSPC/DPPC or DPPC/DPPG SUVs using 3.0M ethanol were 13.7+/-0.7 and 16.7+/-1.7, respectively. The DSPC mole fractions for the DSPC/DPPC SUVs were 0, 0.20, 0.50, 0.67, 0.80 and 1.00, while the DPPG mole fractions were 0, 0.17, 0.50, 0.67, 0.83 and 1.00. Addition of non-interdigitating lipids to the precursor SUVs (e.g., cholesterol or DOPC) inhibited formation of interdigitated sheets. This is consistent with Komatsu and Rowe (Biochem. 30:2463 (1991)), who found that cholesterol inhibited bilayer interdigitation. However, if SUVs containing non-interdigitating lipids were added after the ethanol had been allowed to introduce interdigitation, both components fused in a mixed lipid IFV when the temperature was raised above the Tm (see Figure 29).

The internal volume of the IFVs formed from DPPC/Chol SUVs rapidly decreased as the cholesterol content was increased. The internal volume of the product IFVs was less than 4 µl/µM Pi at 20 mole percent cholesterol. In contrast, adding the cholesterol after the formation of DPPC interdigitated sheets significantly increased the internal volume of the IFVs for a given mole percent of cholesterol. The cholesterol was added in the form of 1:1 mole ratio DPPC/Chol SUVs in 4.0M ethanol. Differential scanning calorimetry of 35 mole percent DPPC/Chol IFVs prepared by either method showed that the DPPC gel-to-liquid crystalline phase transition at 41 deg. C was eliminated in both samples. This demonstrated that cholesterol, which was added after the formation of the DPPC interdigitated sheets, was incorporated into the product DPPC/Chol IFVs.

### EXAMPLE 16

### Incorporation of DPPG into DPPC IF liposomes

IF liposomes were prepared from DPPC/dipalmitoyl phosphatidylglycerol (DPPG) SUVs according to the methods of Example 12 with the following modifications. A total of 30 mM phospholipid (DPPC and DPPG) was employed, 0.17 mole fraction (17 mole percent) DPPG. DPPC and DPPG each in chloroform were added to a round bottom flask (50 ml capacity) and mixed well. The lipids were dried to a thin film in the flask by negative pressure (rotary evaporation) and the resulting film hydrated with 2.0 ml Tris/NaCl buffer, and heated to a temperature of 50-55°C. A total concentration of 3.0 M ethanol was employed. A trace amount of ¹⁴C sucrose was added to the sample, and the suspension sonicated to clarity. Upon removal of the ethanol and incubation of the mixture to 50°-55°C, IF liposomes were formed.

The internal volume of the liposomes was calculated by ¹⁴C sucrose encapsulation and TEMPONE EPR methods according to the methods of Example 13.

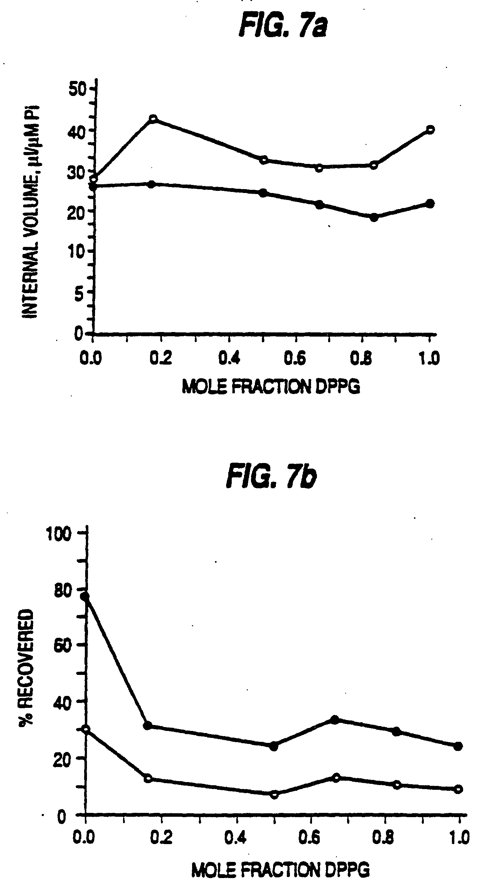

The above methods were repeated with 1.0, 0.83, 0.66, 0.50 and 0.00 mole fraction (100, 83, 66, 50, and 0 mole percent) DPPG. The results are graphically tabulated in Figure 7a and b.

At higher DPPG fractions, IF liposomes were recovered only in low percentages as the liposomes did not pellet well during the wash step, a problem typical of negatively charged liposomes. The internal volumes of the IF liposomes recovered in the pellets are shown in Figure 7a. The open circles are the volumes measured by ¹⁴C encapsulation, while the closed circles are the volumes measured by the broadening agent (TEMPONE) EPR technique. Figure 7b shows the percent recovery of Pi (closed circles) and ¹⁴C labeled sucrose (open circles) as a function of DPPG.

### EXAMPLE 17

### Captured Volume and Encapsulation as a Function of DPPC Initial Concentration

The materials and procedures of Example 12 were followed to form 2.00 ml of DPPC IF liposomes at 20 mg/ml.

The above methods were repeated employing 2.0, 10.0, 20.0, 80.0, and 160.0 mg of DPPC resulting in five samples of 2.00 ml of DPPC IF liposomes at the following DPPC concentrations: 2.5, 5.0, 10.0, 40.0, and 80.0 mg/ml DPPC.

The ¹⁴C sucrose encapsulation percentage and internal volumes of each of the samples were calculated according to the methods of Example 13.

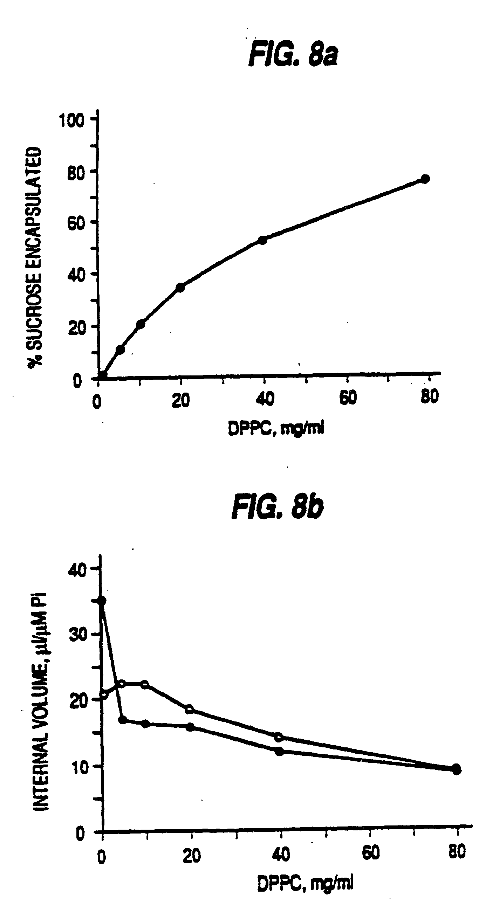

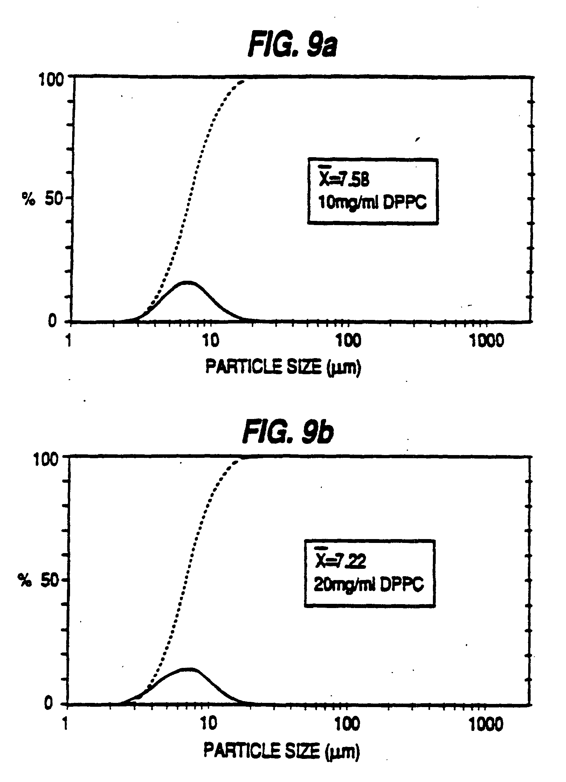

The results are represented graphically in Figure 8a and b. Figure 8a demonstrated that the encapsulation of sucrose increases with the initial DPPC lipid concentration. Figure 8b shows the internal volume of the DPPC IF liposomes measured by both the ¹⁴C sucrose method (open squares) or the EPR method (closed diamonds). The internal volume of the IF liposomes was about 15 to 20 µl/µM Pi when the initial concentration of DPPC was 1 to 20 mg/ml. Measurement made with the Malvern particle sizer indicated that the average diameter of these liposomes (containing 10 mg/ml and 20 mg/ml lipid) is about 7.0-7.5 µm (see Figure 9a and b). This demonstrates that the internal volume of DPPC liposomes formed by the IF method was much larger than conventional MLVs.

### EXAMPLE 18

### Effect of Cholesterol on Formation of DPPC IF Liposomes

The materials and procedures of Example 12 were followed to make IF liposomes employing DPPC and cholesterol, in a total of 30 mM lipid, using a total concentration of 3.0 M ethanol. The cholesterol and DPPC, provided in stock chloroform solutions at concentrations of 20 mg/ml, were admixed in a 50 ml capacity round bottom flask at 95% DPPC and 5% cholesterol. The lipids were dried to a thin film on the surface of the flask, and hydrated with Tris/NaCi as before. The incubation temperature was 50°-55°C.

Aliquots were removed to assay for cholesterol before and after the IF process, to insure the liposomes contained cholesterol, and to assay for the encapsulation of ¹⁴C sucrose.

The above methods were repeated employing 0.00, 0.02, 0.10, 0.15 and 30.0 mole fraction (0, 2.0, 10, 15 and 30 mole percent) of cholesterol.

The internal volumes of the liposomes were determined by ¹⁴C sucrose encapsulation and both the CAT 1 EPR and TEMPONE EPR methods. The cholesterol content of the IF liposomes was measured using o-phthalaldehyde according to the methods of Rudel and Morris, 1973, J. Lipid Res., 14:14.

Results are graphically represented in Figure 10a and b. Figure 10a show the "final" cholesterol concentration of the IF liposomes (open circles) and the final percentage of ¹⁴C sucrose entrapped (open squares). As cholesterol content is increased, the amount of encapsulated sucrose decreased.

Figure 10b shows the decrease in internal volume of the DPPC-cholesterol liposomes as a function of cholesterol content. While the data from the Cat 1 EPR and TEMPONE EPR methods (open triangles and closed circles respectively are in close agreement, the data from the ¹⁴C-sucrose assay shows internal volumes significantly higher. Without being bound to theory, the ¹⁴C-sucrose appears to be "sticking" to the liposomes thus giving readings for higher internal volume.

These studies and Figures 10a and b indicate that the size of the IF liposomes decreases sharply with increasing cholesterol content, a conclusion that was supported by results of Malvern particle sizing; IF liposomes containing 0% cholesterol had an average diameter of 7.66 µm while 30% cholesterol IF liposomes were 3.99 µm.

### EXAMPLE 19

### Effect of Dioleoylphosphatidylcholine (DOPC) on Formation of DPPC IF Liposomes

The materials and procedures of Example 12 were followed to make IF liposomes employing DPPC and dioleoylphosphatidylcholine ("DOPC"), an unsaturated lipid, in a total of 30 mM lipid, using a total concentration of 3.0 M ethanol. The initial liposomes were formed of DPPC and DOPC, provided in stock chloroform solutions at concentrations of 20 mg/ml, which were admixed in a round bottom flask, with 0.20 mole fraction (20 mole percent) DOPC. The lipids were dried to a thin film on the surface of the flask via rotary evaporation, and hydrated with Tris/NaCl as before. The incubation temperature was 50°-55°C.

The above methods were repeated employing 0.00, 0.11, 0.55, 0.72 and 1.00 mole fraction (0, 11, 55, 72, and 100 mole percent) of DOPC.

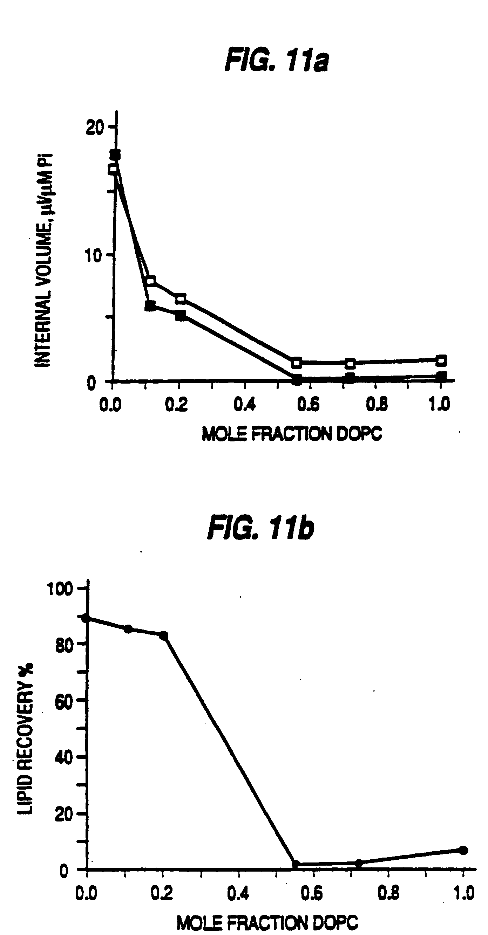

The internal volume of the IF liposomes was measured by the ¹⁴C sucrose encapsulation method and the TEMPONE EPR method, and the results are demonstrated graphically in Figure 11 A.

As can be seen from the graph, increasing the amount of DOPC decreased the size of the IF liposomes. Even as little as 10 % DOPC appeared to reduce the liposome volume by over 50%. Above 0.4 mole fraction (40 mole percent) DOPC the ethanol-lipid gel did not form and the SUVs did not appear to fuse. The internal volumes of the liposomes at 0.6 and 0.8 mole fraction DOPC were 0.2 and 0.24 µl/µM Pi respectively which is in the SUV range. In addition, the percent of lipids recoverable by centrifugation decreased above 0.4 mole fraction DOPC (see Figure 11 B).

### EXAMPLE 20

### Entrapment of Radiocontrast Agent Ioversol in DSPC IF Liposomes

DSPC (200 mg, lyophilized powder) was suspended in 5.0 ml distilled water and sonicated to a translucent SUV suspension (time of sonication was about 20 minutes). The SUV suspension was centrifuged for 10 minutes at 10,000 x g to pellet the titanium residue; SUVs were decanted from the titanium pellet. Ioversol (Optiray 320^{R}, Mallinckrodt) (11.5 mg), 4.1 ml ethanol, and 1.7 ml distilled water were admixed and 3.3 ml aliquots of this mixture were pipetted into three 15 ml capacity Corex^{R} tubes. Aliquots (1.1 ml) of the SUV suspension were added to each tube. The tubes were capped and vortexed vigorously, resulting in a translucent gel.

The tubes were allowed to set at room temperature for one hour, then uncapped and incubated at 70°C in an immersion bath for one hour with intermittent vortical mixing. Following the incubation, the tubes were N₂ sparged by bubbling a gentle stream of N₂ through the mixture for about 8 minutes. After cooling the contents to room temperature, buffer (30 mM Tris, 150 mM NaCl, 0.6 mM Na₂EDTA, pH 6.7) was added to each tube and mixed by inversion. The unentrapped ioversol was removed by centrifugation washes (3 minutes at 5,000 x g) which were repeated 3 times.

The resulting ioversol entrapped in the IF liposomes was assayed spectrophotometrically by absorption at 245 nm and regression against a standard curve of ioversol in ethanol. Lipid concentration was determined by the method of Chen et al. The entrapment results are shown below in Table 6.

**Table 6**

| Sample | mg/ml iodine | mg/ml DSPC | Final Iodine:Lipid |
|---|---|---|---|
| 1 | 92.1 +/- 7.1 | 14.3 +/- 0.2 | 6.4 (range 5.9-7.0) |
| 2 | 93.2 +/- 8.1 | 13.7 +/- 0.7 | 6.8 (range 5.9-7.8) |
| 3 | 93.2 +/- 9.2 | 13.7 +/- 0.1 | 6.8 (range 6.1-7.5) |

The IF liposomes had a mean diameter of 4.0-5.0 µm determined by Malvern particle size analysis.

### EXAMPLE 21

### Entrapment of Radiocontrast Agent Ioxoglate in DSPC IF Liposomes

The materials and procedures of Example 20 were followed thereby entrapping the radiocontrast agent ioxoglate (Hexabrix^{R}, Mallinckrodt) in IF liposomes. Entrapment was assayed according to the methods of Example 20 and the results tabulated in Table 7 below.

**Table 7**

| Contrast Agent | mg/ml Iodine | mg/ml Lipid | Iodine:Lipid |
|---|---|---|---|
| Ioxoglate | 64.3 +/- 2.7 | 11.5 +/- 0.3 | 5.2-6.0 |

### EXAMPLE 22

### Entrapment of Radiocontrast Agent Iopamidol in DSPC IF Liposomes

The materials and procedures of Example 20 were followed thereby entrapping the radiocontrast agent iopamidol (Isovue^{R}, Bristol-Myers Squibb) in IF liposomes. Entrapment was assayed according to the methods of Example 20 and the results tabulated in Table 8 below.

**Table 8**

| Contrast Agent | mg/ml Iodine | mg/ml Lipid | Iodine:Lipid |
|---|---|---|---|
| Iopamidol | 52.3 +/- 5.2 | 12.0 +/- 0.6 | 3.7-5.0 |

### EXAMPLE 23

### Effect of Incubation Time on Internal Volume of IF Liposomes

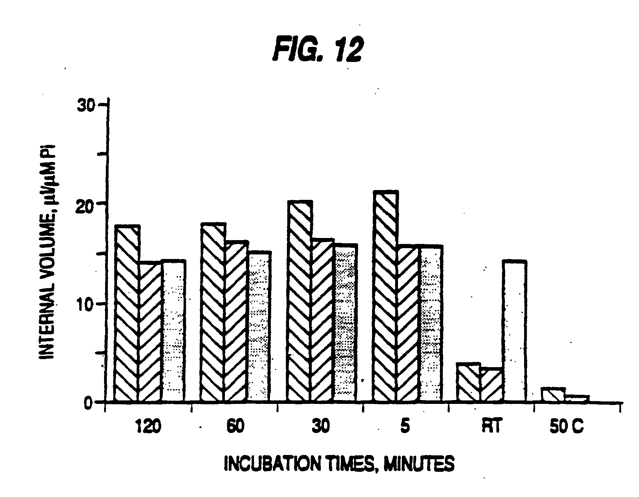

The materials and procedures of Example 12 were followed, using 20 mg/ml DPPC and wherein the ethanol concentration was 3.0 M, wherein the incubation period of the gel at a temperature above and below the Tm was varied. The incubation period was set at 5 minutes, and the internal volume of the resulting IF liposomes was measured by the ¹⁴C sucrose encapsulation method (solid bar), and both the CAT 1 EPR (shaded bar), and TEMPOME EPR method (diagonal bar). The results of the measurements are demonstrated on the histogram of Figure 12.

The above methods were repeated wherein the incubation periods were 30 minutes, one hour, and two hours. Similarly, internal volume measurements were made and compared.

As shown by Figure 12, there appears to be no significant difference in the IF liposome internal volume as a function of incubation time between 5 minutes and 2 hours.

### EXAMPLE 24

### Effect of Changing Incubation Procedure on the Internal Volume of IF Liposomes

The materials and procedures of Example 12 were repeated wherein the incubation conditions were varied, e.g., wherein the DPPC SUVs were incubated at room temperature only, without incubation above the DPPC Tm (e.g., at 50-55°C). The internal volume of the resulting IF liposomes was determined by the ¹⁴ C sucrose encapsulation method (solid bars) as well as both the CAT 1 EPR (shaded bars), and TEMPONE EPR methods (diagonal bars), and the results are shown on the histogram as "RT" of Figure 12.

The above procedure was repeated without room temperature incubation, wherein the ethanol was added and the incubation was conducted at a temperature above the DPPC Tm (50-55°C). Since the liposomes resulting from this method of incubation did not pellet, rather than centrifugation washes, the liposomes were washed by diluting the DPPC sample 6 fold with 10 ml of Tris/NaCl buffer, removing a 4.0 ml sample, and concentrating this sample with an Amicon™ 30K microconcentrator (Grace Co.) which was spun for 1 hour at 30,000 x g in a Beckman J-2 centrifuge. The sample volume which was retained was concentrated again using the same procedure. The results of this sample are labeled "50°C".

By reference to Figure 12, it is apparent that both incubation periods are required for the formation of large IF liposomes.

### EXAMPLE 25

Comparison of Internal Volume of IF Liposomes to MLVs

### I

DPPC (80 mg, powdered form) (Avanti Polar Lipids) was added to 2.00 ml of Tris/NaCl buffer according to the methods of Example 12. The DPPC suspension was sonicated to clarity as described in Example 12. A final concentration of 3.0 M ethanol (addition of 0.43 ml ethanol) was employed thereby forming IF liposomes. The sample was incubated and washed by centrifugation as in Example 12, and the internal volume was determined twice by the CAT 1 EPR methods described in Example 13, the results tabulated in Table 9 hereinbelow.

### II

The above methods of section I were repeated employing 80 mg DPPC in 2.00 ml Tris/NaCl buffer, vortexing at 50-55°C thereby producing MLVs. However, these MLVs were not sonicated and no ethanol was added.

The identical methods were repeated wherein 40 mg of DPPC was employed. The sample was incubated and washed by centrifugation as in Example 12, and the internal volume was determined twice by the CAT 1 EPR methods described in Example 13, the results tabulated in Table 9 hereinbelow.

### III

The methods of section II were repeated wherein 0.43 ml of ethanol for a final concentration of 3.0 M ethanol in the final solution was added. The sample was incubated and washed by centrifugation as in Example 12, and the internal volume was determined twice by the CAT 1 EPR methods described in Example 13, the results tabulated in Table 9 hereinbelow.

Table 9 below illustrates the relatively large internal volume of IF liposomes compared with conventional MLVs or MLVs exposed to relatively high ethanol concentrations.

**Table 9**

| Sample | Lipid Concentration (mg/ml) | 3.0 M EtOH | Internal Volume (µL/µM Pi) |
|---|---|---|---|
| | | | |
| DPPC SUVs | 20 | yes | 15.78 |
| DPPC SUVs | 40 | yes | 13.38 |
| | | | |
| DPPC MLVs | 20 | yes | 2.02 |
| DPPC MLVs | 40 | yes | 1.70 |
| | | | |
| DPPC MLVs | 20 | no | 1.37 |
| DPPC MLVs | 40 | no | 0.92 |

### EXAMPLE 26

### Iotrolan-DSPC IF Liposomes

The materials and procedures of Example 7 were followed, thus producing an IF liposome population containing iotrolan.

### EXAMPLE 27

### Entrapment of Radiocontrast Agent Iopromide in DSPC IF Liposomes

The materials and procedures of Example 22 are followed thus producing IF liposomes containing iopromide.

### EXAMPLE 28

### Pressure Induced Interdigitation Fusion Liposomes

Interdigitation fusion liposomes were made using hydrostatic pressure to induce the fusion. These are referred to herein as pressure induced fusion liposomes or "PIFs". Small liposomes were made by probe sonicating either 20 mg/ml DPPC MLVs or DSPC MLVs uncil clear. The resulting small unilamellar vesicles (SUVs) were centrifuged at 3000g for 5 minutes to remove titanium dust introduced from the probe sonicator. Two to three ml of the SUVs was placed in a Teflon® polytetrafluoroethylene sample holder which was then submerged in the hydraulic fluid inside the high pressure reaction chamber. The temperature of the reaction chamber was brought co the desired value before the sample was placed in it. To vary temperature the reaction chamber was jacketed with flexible tubing through which water circulated from a water bath. Once the sample was loaded the chamber was then closed and hydraulic fluid pumped in to pressurize the sample. Samples were held at pressure for 15 minutes after which pressure was reduced and the sample removed. Sample was then transferred from the Teflon® holder to a glass vial and heated to 50°C (for DPPC) or 70°C (for DSPC) to ensure reformation of vesicle structure. Liposomes (now PIFs) were allowed to cool and captured volumes measured using a technique described in detail in Perkins, ec al. (1988) Biochim. Biophys. Acta 943: 103-107.

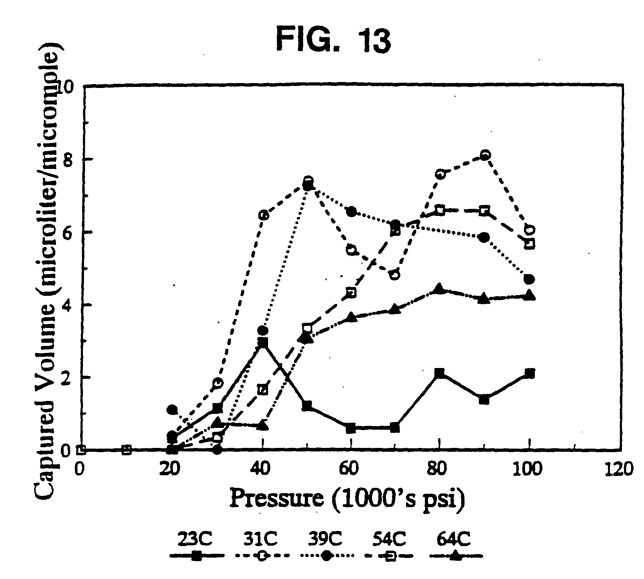

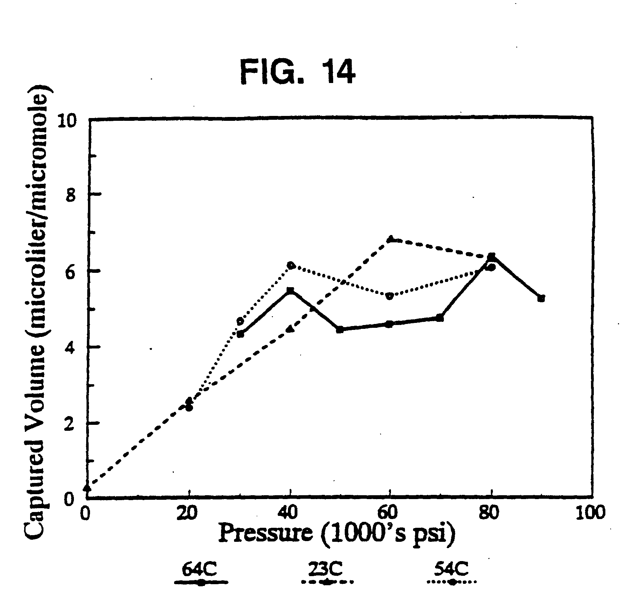

The results are presented graphically in Figure 13 for the DPPC tests, and in Figure 14 for the DSPC tests. For DPPC, as shown in Figure 13, samples pressurized below the phase transition temperature of the lipid (41°C for DPPC) were gel like in appearance -- just like the gels resulting from ethanol addition below the phase transition temperature. The viscous nature of these samples disappeared after they were heated above 41°C. For DSPC, as shown in Figure 14, samples pressurized below the phase transition temperature of the lipid (54°C for DSPC) were gel like in appearance. The viscous nature of these samples disappeared after they were heated to 70°C. In both cases, heating resulted in transformation from interdigitated sheets to liposomes.

### Example 29

### High Pressure Sterilization

Vegetative bacteria, specifically those of the genus *Bacillus* are known to be extremely resistant to a variety of environmental stresses including high temperatures and pressures. Indeed it has long been known that pressures higher than 1000 MPa are needed to inactivate bacterial spores (Larson, *et al,:* The Effect of High Pressure on Bacteria, J. Infectious Diseases, 22, 271-279 (1918)). Therefore, spore suspensions of *Bacillus subtilis* ATCC strain 6633 were plated on blood agar (Remel) and used as the seed culture for all of the sterilization studies. Colonies from the agar were grown in trypticase soy broth (Remel) to the early stationary phase. These cultures contained 7.7 X 10⁷ viable bacteria per milliliter, as measured by growth on blood agar.

Cultures that were subjected to high pressures were placed in Teflon® polytetrafluoroethylene tubes with screw caps (Swage Lok) and placed in the high pressure reactor chamber. The pressure on the reactor was then increased to the desired point (High Pressure Equip. Co., PS 150 pumping system) and time at that pressure was then monitored. At the completion of the incubation, the sample was plated on blood agar, and viable colonies were counted after 15 hours of growth.

Figures 15, 16 and 17 show the effects of high pressures on *B*. *subtilis* at 40, 50 and 60°C respectively. Each data point is the average of at least 3 to 9 separate determinations. Error bars are not shown because they are usually smaller than the symbol used to denote the point. Sterility is determined by the lack of growth on blood agar. On the graphs, two consecutive points with a value of 0 show the time required to confidently reach sterility. At 40°C sterility could be achieved only after 90 minutes at 80,000 psi. This time is reduced to 30 minutes at 50°C. Although strictly speaking the log of 0 is undefined, for the purposes of presenting the present data, the log of 0 has been graphed as 0.

In order to ascertain that the presence of lipid has no protective effect on the bacteria, and that the presence of bacteria do not affect the formation of the interdigitated phase, samples of DPPC SUVs were mixed with 100,000 viable bacteria. The mixture was then subjected to sterilizing pressures. Sterilization was found to be effective, and the captured volume of the resultant PIF liposomes was not affected by the presence of bacteria. Therefore, these results indicate that high hydrostatic pressures may be of use in sterilizing liposomal preparations.

Further tests were conducted to demonstrate that high pressure may be used to sterilize liposomal products other than those involving pressure induced fusion vesicles. Multilamellar egg phosphatidylcholine (EPC) liposomes containing gentamicin were formed in accordance with the methods set forth in Lenk et al., U.S Patent No. 4,522,803, A sample of this material was subjected to 6204,6 x 10⁵ Pa (90000 psi) pressure for 60 minutes at a temperature of 50°C. This sample, as well as an untreated control sample were then centrifuged to separate free from bound gentamicin. The supernatants were assayed to determine free gentamicin, ant the liposomes were resuspended and assayed for liposomal associated gentamicin. The unpressurized control sample was found to have 0.723 mg/ml of non-liposomally associated gentamicin, and 6.687 mg/ml of liposomally associated gentamicin. The pressurized test sample had corresponding values of 0.744 mg/ml of non-liposomally associated gentamicin, and 7.234 mg/ml of liposomally associated gentamicin. The data indicate that these pressure and temperature conditions do not adversely affect the association of gentamicin with EPC liposomes.

### Example 30

### Post Gel Incorporation of Lipid-Soluble Molecules into Interdigitation-Fusion Liposomes

The interdigitation-fusion (IF) technique can also be applied to SUVs composed of two or more lipid components, rather than the single lipid component systems. The IF technique is very effective at producing large liposomes if both lipids in the SUVs can undergo ethanol-induced interdigitation. For example, large incerdigitation-fusion vesicles (IFVs) were produced from either DPPC/DMPC SUVs or DPPC/DPPG SUVs, if the temperature was such that both lipid components undergo ethanol-induced interdigitation.

The internal volume results from the DPPC/DPPG SUV mixture are shown in Figure 13. As shown in the figure the internal volume of IFVs formed from DPPC/DPPG SUVs did not change significantly as the mole fraction DPPG was increased. The IF technique as described in Example 1 was used to form these IFVs, and the total lipid concentration for each experiment was 30 mM. The ethanol-induced DPPC/DPPG gel state was formed at room temperature. The internal volume of the product IFVs were measured with both ¹⁴C-sucrose encapsulation and the EPR probe TEMPONE, as previously described.

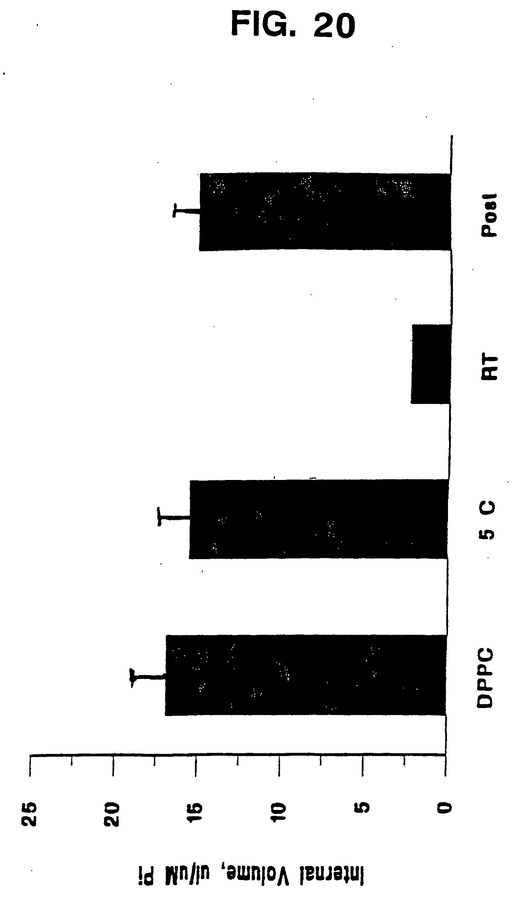

In contrast to the DPPC/DPPG SUV experiment, the addition of a non-interdigitating lipid such as DOPC or cholesterol to DPPC SUVs significantly inhibited the formation of large IFVs. The internal volume of IFVs formed from DPPC/DOPC and DPPC/cholesterol SUVs are shown as a function or mole fraction DOPC or cholesterol in Figure 20. The internal volumes of the product liposomes were measured using the EPR probe TEMPONE. The internal volume of the product liposomes were reduced by 50% when the DOPC content in the DPPC/DOPC SUVs reached 10 mole percent. SUVs that were over 50 mole percent DOPC did not fuse to form the interdigitated membrane sheets required for IFV formation. Cholesterol was particularly effective at disrupting the IF technique. The IFV internal volume was significantly reduced even for DPPC/cholesterol SUVs which contained as little as 2 to 5 mole percent cholesterol.

Therefore, the effectiveness of the IF technique for producing high internal volume liposomes was significantly reduced when non-interdigitating lipids were included in the SUVs prior to interdigitation fusion. Non-interdigitating lipids, such as DOPC and cholesterol, inhibited the ethanol-induced bilayer interdigitation which is required to induce SUV fusion and formation of the interdigitated membrane sheets. This effect presents a problem for preparing large IFVs which have lipids which do not interdigitate.

However, in accordance with the present invention, a modification of the basic IF technique was developed to more efficiently introduce non-interdigitating lipids into IFVs. This IF technique modification comprises adding the non-interdigitating lipid after the ethanol-induced interdigitated membrane sheets or "gel" state is formed. Typically, SUVs containing the non-interdigitating lipids are mixed into the ethanol/phospholipid gel prior to raising the temperature above Tm. The SUVs which contain the non-interdigitating lipids fuse with the phospholipid sheets when the temperature is raised above the Tm of the phospholipid membrane sheets. This IF technique modification will be referred to herein as "post-gel" incorporation, because the extra lipids were added after the ethanol/phospholipid "gel" state was formed.

In addition, this post-gel incorporation technique can be used to incorporate into IF vesicles any material which would normally interfere with the IF process. That is, certain materials which it may be desirable to incorporate into IF vesicles, such as bioactive materials, may interfere with the IF process, and therefore not be otherwise usable. The present post-gel incorporation process allows the incorporation of these interdigitation-fusion interfering materials into IF vesicles. This process is particularly suited for hydrophobic or amphipathic materials which are lipid soluble. Bioactive materials which may desirably incorporated into IF vesicles are described elsewhere in this specification.

Even though DMPC is a saturated phospholipid, incorporation of DMPC SUVs into an DPPC IFVs at room temperature is a good example of the post-gel lipid incorporation. Since the Tm for DMPC is 23°C, ethanol will not induce interdigitation in DMPC bilayers at room temperature. DMPC prevented the formation of large IFVs when the ethanol was added to DPPC/DMPC SUVs at room temperature. However, large IFVs were formed when ethanol was added to DPPC/DMPC SUVs at 5°C. Thus mixing in DMPC to DPPC SUVs inhibited the IF technique at room temperature, but not at 5°C. However, relatively large DPPC/DMPC IFVs were formed at room temperature even at 1:1 DPPC/DMPC mole ratio if the DMPC was added to the sample after the formation of the ethanol/DPPC gel state.

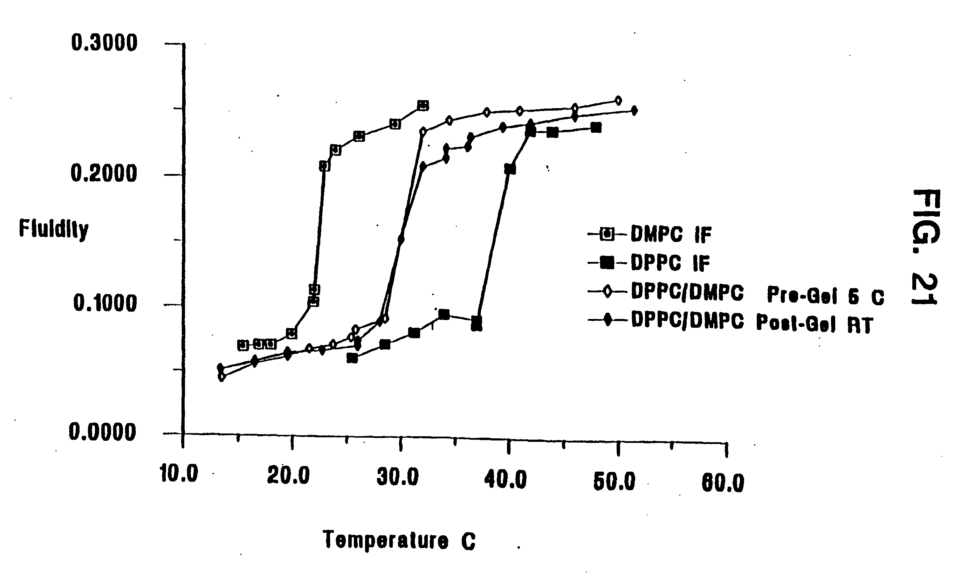

Evidence for post-gel state mixing of DMPC SUVs into DPPC IFVs is shown in Figures 20 and 21. Figure 20 shows the average internal volume for pure DPPC IFVs which were formed according the procedures described in the earlier examples. In addition, DPPC/DMPC 1:1 mole ratio IFVs which were formed following the three different modified versions of the standard IF technique. The bar labeled "5°C" and "RT" indicate the internal volume of IFVs which formed from DPPC/DMPC 1:1 mole ratio SUVs in which the ethanol/lipid gel state was formed at 5°C and room temperature respectively. The bar labeled "post" indicates the internal volume of DPPC/DMPC 1:1 mole ratio IFVs which were formed at room temperature using the post-gel incorporation modification.

As shown by the results presented in Figure 20, the addition of DMPC SUVs to the ethanol-induced DPPC gel state did not inhibit the formation of large IFVs. In order to demonstrate that the DMPC was incorporated into the IFVs, the phase transition behavior of the DPPC/DMPC IFVs was examined. Figure 21 shows the membrane fluidity of DPPC, DMPC, and DPPC/DMPC IFVs as a function of temperature. The membrane fluidity was measured with the EPR probe TEMPO according to the method of Wu and McConnell. The gel state formation temperatures for the DPPC and DMPC IFVs were at room temperature and 5°C respectively. The DPPC/DMPC curve labeled "5°C" was formed from DPPC/DMPC at 1:1 mole ratio SUVs at 5°C. The DMPC for the DPPC/DMPC curve labeled "post" was added at room temperature as DMPC SUVs after the ethanol-induced DPPC gel state was formed. The main phase transition temperatures for the DMPC and DPPC IFVs were at 23 and 39°C respectively. The Tm for DPPC IFVs was 39°C which is slightly below the established Tm for DPPC. DPPC and DMPC are completely miscible in both the gel and liquid-crystalline phases, forming an ideal mixture. The phase transition temperature for DPPC/DMPC 1:1 mole ratio IFVs for which the gel was formed from 1:1 mole ratio DPPC/DMPC SUVs at 5°C was at 30°C which is consistent with established values for 1:1 mole ratio DPPC/DMPC MLVs. The Tm for the DPPC/DMPC IFVs that were formed by the post-gel IF modification was also at 30°C which indicates that the mole ratio of these DPPC/DMPC IFVs was also 1:1. This demonstrates that most of the DMPC SUVs were incorporated into the DPPC IFVs during the 50-55°C incubation.

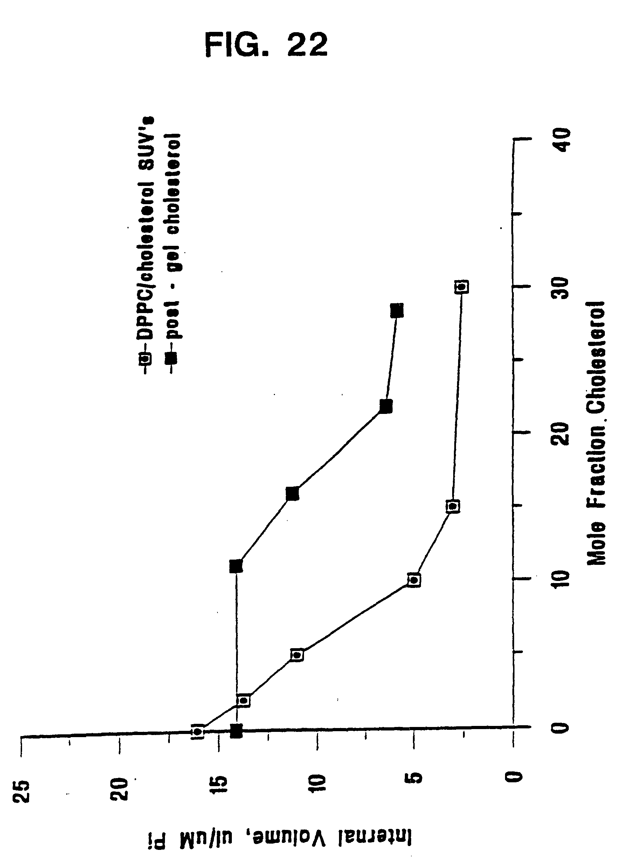

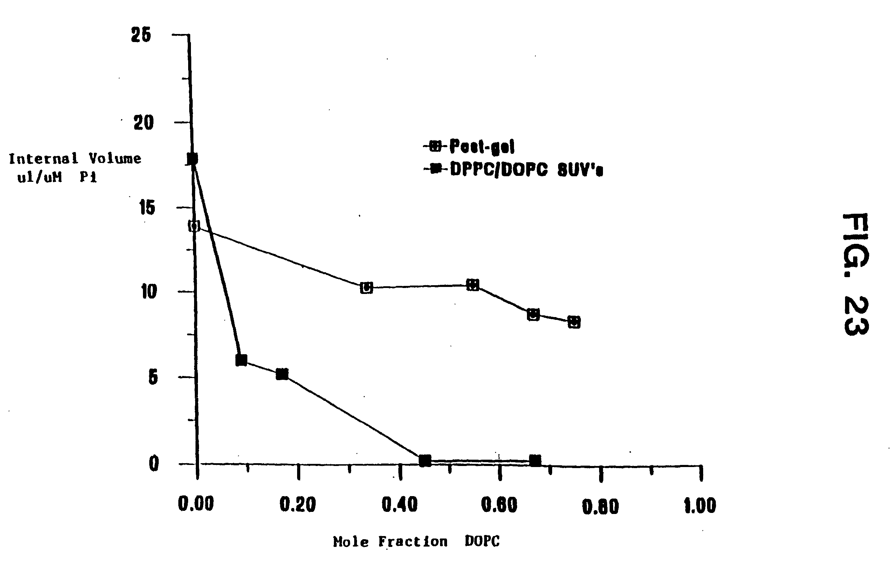

Non-interdigitating lipids that do not mix in an ideal manner can also be mixed into DPPC IFVs. Figure 22 shows the internal volume of DPPC cholesterol IFVs that were formed by mixing the cholesterol into the sample after the ethanol-induced DPPC gel state was formed. The internal volume as a function of mole fraction cholesterol recovered in the product DPPC/cholesterol IFVs is shown in this figure. The cholesterol content in the IFVs was determined using radiolabeled cholesterol. For comparison the internal volumes of DPPC/cholesterol IFVs formed form DPPC/cholesterol SUVs are also shown in Figure 22. Figure 23 shows results for a similar experiment where the non-interdigitating lipid DOPC was mixed into DPPC IFVs using either the post-gel IF modification or the standard IF technique using DPPC/DOPC SUVs. The DOPC content was determined using radiolabeled DOPC. This figure illustrates that incorporating non-interdigitating lipids into the sample after the interdigitated membrane sheet were formed significantly increased the internal volume of mixed lipid IFVs.

Typical second lipids which may be able to be incorporated into interdigitation-fusion liposomes formed of a first lipid by this post-gel incorporation method include, but are not limited to, cholesterols, tocopherols, egg PC, POPC, DOPC, DMPC, DPPE, egg PE and fatty acids. These are all lipids which are either non-interdigitating or which have a transition temperature (Tm) below room temperature. By this method, as much as 90% of these lipids may be incorporated into IFVs formed of an interdigitating lipid. Therefore the weight ratio of the first lipid to the second lipid may be as low as 10:90. On the other hand, the amount of the second lipid which may interfere with the interdigitation-fusion process may be as low as 0.1% of the first lipid. Therefore the ratio of the first lipid to the second lipid may be as high as 99.9:0.1.

## Claims

1. A method of making interdigitation-fusion liposomes which comprises the steps of :
(a) interdigitating-fusing sized liposomes comprising a symmetrical saturated acyl chain phospholipid with an amount of an inducer effective to fuse the liposomes ;
(b) incubating the composition of step (a) at a temperature which is below the transition temperature of the composition for a period of time effective to form an interdigitation-fusion gel from the composition, wherein the period of time is from 1 minute to 1 hour ;
(c) adding a material which would interfere with the interdigitation-fusing step (a) to said gel of step (b)to form a mixture and ;
(d) incubating the mixture of step (c) at a temperature which is greater than the transition temperature of the phospholipid in the gel so as to form interdigitation-fusion liposomes comprising the material from said gel.

2. The method of claim 1, wherein the symmetrical saturated acyl chain phospholipid is selected from the group consisting of :
dimyristoylphosphatidylcholine,
distearoylphosphatidylcholine,
dipalmitoylphosphatidylcholine,
dimyristoylphosphatidylserine,
dipalmitoylphosphatidylserine,
distearoylphosphatidylserine,
dimyristoylphosphatidylethanolamine,
dipalmitoylphosphatidylethanolamine,
distearoylphosphatidylethanolamine,
dimyristoylphosphatidic acid,
distearoylphosphatidic acid,
dipalmitoylphosphatidic acid,
dimyristoylphosphatidylinositol,
distearoylphosphatidylinositol,
dipalmitoylphosphatidylinositol,
hydrogenated soy phosphatidylcholine,
hydrogenated soy lecithin,
dipalmitoylphosphatidylglycerol,
di-0-hexadecylphosphatidylcholine
distearoylphosphatidylglycerol, and
dimyristoylphosphatidylglycerol.

3. The method of claim 1, wherein the liposomes of step (a) have a size of less than 0.050 µm (micron).

4. The method of claim 3, wherein the liposomes of step (a) have a size of 0.025 µm (micron).

5. The method of any of claims 1 to 4, wherein the material of step (c) is a bioactive agent.

6. A method of making interdigitation-fusion liposomes comprising:
a) interdigitating-fusing sized liposomes comprising at least one symmetrical saturated acyl chain phospholipid in the presence of an amount of an inducer effective to fuse said liposomes,
b) incubating the composition of step a) at a temperature below the transition temperature of the composition for a period ranging from 1 minute to 1 hour to fuse said liposomes into an interdigitation-fusion gel comprising phospholipid membrane sheets,
c) adding an additional lipid to said gel to form a mixture, said additional lipid being non-interdigitating or having a transition temperature below room temperature,
d) incubating said mixture at a temperature which is greater than the transition temperature of the phospholipid membrane sheets in the gel as to form interdigitation-fusion liposomes comprising both lipids.

7. The method of claim 6, wherein the aditional lipid is a noninterdigitating lipid.

8. The method of claim 7, wherein the lipid is selected from the group consisting of :
cholesterols,
tocopherols,
egg phosphatidylcholine,
palmitoyloleoylphosphatidylcholine, and
dioleoylphosphatidylcholine.

## Patentansprüche

1. Verfahren zur Herstellung von Interdigitations-Fusionsliposomen, welches die Schritte umfasst:
(a) Interdigitationsfusionieren klassierter Liposomen, die ein symmetrisches Phospholipid mit gesättigten Acylketten umfassen, mit einer Menge an Induktor, die wirksam ist, um die Liposomen zu fusionieren;
(b) Inkubieren der Zusammensetzung von Schritt (a) bei einer Temperatur, die unterhalb der Umwandlungstemperatur der Zusammensetzung liegt, über eine Zeitspanne, die wirksam ist, um aus der Zusammensetzung ein Interdigitations-Fusionsgel zu bilden, wobei die Zeitspanne 1 Minute bis 1 Stunde beträgt;
(c) Zugabe eines Materials, das den Interdigitations-Fusionsschritt (a) stören würde, zu dem Gel von Schritt (b), um eine Mischung zu bilden; und
(d) Inkubieren der Mischung von Schritt (c) bei einer Temperatur, die größer ist als die Umwandlungstemperatur des Phospholipids in dem Gel, um Interdigitations-Fusionsliposomen, die das Material umfassen, aus dem Gel zu bilden.

2. Verfahren nach Anspruch 1, in dem das symmetrische Phospholipid mit gesättigten Acylketten ausgewählt ist aus der Gruppe bestehend aus:
Dimyristoylphosphatidylcholin,
Distearoylphosphatidylcholin,
Dipalmitoylphosphatidylcholin,
Dimyristoylphosphatidylserin,
Dipalmitoylphosphatidylserin,
Distearoylphosphatidylserin,
Dimyristoylphosphatidylethanolamin,
Dipalmitoylphosphatidylethanolamin,
Distearoylphosphatidylethanolamin,
Dimyristoylphosphatidsäure,
Distearyolyphosphatidsäure,
Dipalmitoylphosphatidsäure,
Dimyristoylphosphatidylinosit,
Distearoylphosphatidylinosit,
Dipalmitoylphosphatidylinosit, hydriertes Sojaphosphatidylcholin, hydriertes Sojalecithin,
Dipalmitoylphosphatidylglycerin,
Di-O-hexadecylphosphatidylcholin,
Distearoylphosphatidylglycerin und
Dimyristoylphosphatidylglycerin.

3. Verfahren nach Anspruch 1, in dem die Liposomen von Schritt (a) eine Größe von weniger als 0,050 µm (Mikrometer) aufweisen.

4. Verfahren nach Anspruch 3, in dem die Liposomen von Schritt (a) eine Größe von 0,025 µm (Mikrometer) aufweisen.

5. Verfahren nach irgendeinem der Ansprüche 1 bis 4, in dem das Material von Schritt (c) ein bioaktives Mittel ist.

6. Verfahren zur Herstellung von Interdigitations-Fusionsliposomen, umfassend:
(a) Interdigitationsfusionieren klassierter Liposomen, die mindestens ein symmetrisches Phospholipid mit gesättigten Acylketten umfassen, in Anwesenheit einer Menge eines Induktors, die wirksam ist, um die Liposomen zu fusionieren,
(b) Inkubieren der Zusammensetzung von Schritt (a) bei einer Temperatur unterhalb der Umwandlungstemperatur der Zusammensetzung über eine Zeitspanne im Bereich von 1 Minute bis 1 Stunde, um die Liposomen zu einem Interdigitations-Fusionsgel zu fusionieren, das Phospholipid-Membranlagen umfasst,
(c) Zugabe eines zusätzlichen Lipids zu dem Gel, um eine Mischung zu bilden, wobei das zusätzliche Lipid keine Interdigitation eingeht oder eine Umwandlungstemperatur unterhalb von Raumtemperatur aufweist,
(d) Inkubieren der Mischung bei einer Temperatur, die größer ist als die Umwandlungstemperatur der Phospholipid-Membranlagen in dem Gel, um Interdigitations-Fusionsliposomen zu bilden, die beide Lipide umfassen.

7. Verfahren nach Anspruch 6, in dem das zusätzliche Lipid ein Lipid ist, das keine Interdigitation eingeht.

8. Verfahren nach Anspruch 7, in dem das Lipid ausgewählt ist aus der Gruppe bestehend aus:
Cholesterolen,
Tocopherolen,
Ei-Phosphatidylcholin,
Palmitoyloleoylphosphatidylcholin und
Dioleoylphosphatidylcholin.

## Revendications

1. Procédé de préparation de liposomes d'interdigitation-fusion, comprenant les étapes suivantes :
(a) interdigitation-fusion de liposomes de taille choisie, comprenant un phospholipide à chaîne acyle saturée symétrique, avec une quantité d'un inducteur efficace pour faire fusionner les liposomes ;
(b) incubation de la composition de l'étape (a) à une température qui est inférieure à la température de transition de la composition, pendant une durée efficace pour former un gel d'interdigitation-fusion à partir de la composition, la durée allant de 1 minute à 1 heure ;
(c) addition audit gel de l'étape (b) d'une substance interférant avec l'étape (a) d'interdigitation-fusion, pour former un mélange et
(d) incubation du mélange de l'étape (c) à une température qui est supérieure à la température de transition du phospholipide dans le gel, de manière à former des liposomes d'interdigitation-fusion comprenant la substance provenant dudit gel.

2. Procédé de la revendication 1, dans lequel le phospholipide à chaîne acyle saturée symétrique est choisi dans le groupe constitué par :
la dimyristoylphosphatidylcholine,
la distéaroylphosphatidylcholine,
la dipalmitoylphosphatidylcholine,
la dimyristoylphosphatidylsérine,
la dipalmitoylphosphatidylsérine,
la distéaroylphosphatidylsérine,
la dimyristoylphosphatidyléthanolamine,
la dipalmitoylphosphatidyléthanolamine,
la distéaroylphosphatidyléthanolamine,
l'acide dimyristoylphosphatidique,
l'acide distéaroylphosphatidique,
l'acide dipalmitoylphosphatidique,
le dimyristoylphosphatidylinositol,
le distéaroylphosphatidylinositol,
le dipalmitoylphosphatidylinositol,
la phosphatidylcholine de soja hydrogénée,
la lécithine de soja hydrogénée,
le dipalmitoylphosphatidylglycérol,
la di-O-hexadécylphosphatidylcholine,
le distéaroylphosphatidylglycérol et
le dimyristoylphosphatidylglycérol.

3. Procédé de la revendication 1, dans lequel les liposomes de l'étape (a) ont une taille inférieure à 0,050 µm (micromètre).

4. Procédé de la revendication 3, dans lequel les liposomes de l'étape (a) ont une taille de 0,025 µm (micromètre).

5. Procédé de l'une quelconque des revendications 1 à 4, dans lequel la substance de l'étape (c) est un agent bioactif.

6. Procédé de préparation de liposomes d'interdigitation-fusion, comprenant les étapes suivantes :
(a) interdigitation-fusion de liposomes de taille choisie, comprenant au moins un phospholipide à chaîne acyle saturée symétrique, en présence d'une quantité d'un inducteur efficace pour faire fusionner les liposomes ;
(b) incubation de la composition de l'étape (a) à une température qui est inférieure à la température de transition de la composition, pendant une durée allant de 1 minute à 1 heure, pour faire fusionner les liposomes en un gel d'interdigitation-fusion comprenant des feuillets de membrane phospholipidique ;
(c) addition audit gel d'un lipide supplémentaire pour former un mélange, ledit lipide supplémentaire étant inapte à l'interdigitation ou ayant une température de transition inférieure à la température ambiante ;
(d) incubation dudit mélange à une température qui est supérieure à la température de transition des feuillets de membrane phospholipidique dans le gel, de manière à former des liposomes d'interdigitation-fusion comprenant les deux lipides.

7. Procédé de la revendication 6, dans lequel le lipide supplémentaire est un lipide inapte à l'interdigitation.

8. Procédé de la revendication 7, dans lequel le lipide est choisi dans le groupe constitué par :
les cholestérols,
les tocophérols,
la phosphatidylcholine d'oeuf,
la palmitoyloléoylphosphatidylcholine et
la dioléoylphosphatidylcholine.
